# EUROPEAN PATENT SPECIFICATION

(11) **EP 2 806 892 B1**
(45) Date of publication and mention of the grant of the patent: **06.03.2019**
(21) Application number: 13701871.9
(22) Date of filing: 25.01.2013
(51) Int. Cl.: A61K 39/395, C12N 9/24, C07K 16/32

(54) **COMBINED THERAPEUTIC USE OF ANTIBODIES AND ENDOGLYCOSIDASES**
KOMBINATIONSTHERAPIE MIT ANTIKÖRPERN UND ENDOGLYKOSIDASEN
TRAITEMENT COMBINÉ AVEC DES ANTICORPS ET DES ENDOGLYCOSIDASES

(30) Priority: 26.01.2012 GB 201201314
(43) Date of publication of application: 03.12.2014
(73) Proprietor: Immago Biosystems Ltd, Cheltenham GL52 6UE (GB)
(72) Inventor: CRISPIN, Matthew David Max, Cheltenham GL52 6UE (GB); SCANLAN, Christopher Neil, deceased (GB)
(74) Representative: J A Kemp
(86) International application number: PCT/GB2013/050164
(87) International publication number: WO 2013/110946

(56) References cited:
- WO-A2-2008/071418
- WO-A2-2009/033670
- NANDAKUMAR ET AL: "Therapeutic cleavage of IgG: new avenues for treating inflammation", TRENDS IN IMMUNOLOGY, ELSEVIER LTD. * TRENDS JOURNALS, GB, vol. 29, no. 4, 6 March 2008 (2008-03-06), pages 173-178, XP022575981, ISSN: 1471-4906
- KAVITHA BARUAH ET AL: "Selective Deactivation of Serum IgG: A General Strategy for the Enhancement of Monoclonal Antibody Receptor Interactions", JOURNAL OF MOLECULAR BIOLOGY, ACADEMIC PRESS, UNITED KINGDOM, vol. 420, no. 1, 3 April 2012 (2012-04-03) , pages 1-7, XP028514772, ISSN: 0022-2836, DOI: 10.1016/J.JMB.2012.04.002 [retrieved on 2012-04-05]
- ALBERT HEIKE ET AL: "In vivo enzymatic modulation of IgG glycosylation inhibits autoimmune disease in an IgG subclass-dependent manner", PROCEEDINGS OF THE NATIONAL ACADEMY OF SCIENCES, NATIONAL ACADEMY OF SCIENCES, US, vol. 105, no. 39, 30 September 2008 (2008-09-30), pages 15005-15009, XP002569095, ISSN: 0027-8424, DOI: 10.1073/PNAS.0808248105 [retrieved on 2008-09-24]
- ALLHORN M ET AL: "Human IgC/Fc[gamma]R interactions are modulated by streptococcal IgG glycan hydrolysis", PLOS ONE, PUBLIC LIBRARY OF SCIENCE, US, vol. 3, no. 1, 9 January 2008 (2008-01-09) , pages e1413.1-e1413.12, XP002508160, ISSN: 1932-6203, DOI: 10.1371/JOURNAL.PONE.0001413
- YADONG WEI ET AL: "Glycoengineering of Human IgG1-Fc through Combined Yeast Expression and in Vitro Chemoenzymatic Glycosylation +", BIOCHEMISTRY, vol. 47, no. 39, 30 September 2008 (2008-09-30), pages 10294-10304, XP055059067, ISSN: 0006-2960, DOI: 10.1021/bi800874y

### SEQUENCE LISTING

<110> CRISPIN, Matthew David Max SCANLAN, Christopher Neil
<120> Antibody composition
<130> 489.114439/01
<160> 2
<170> PatentIn version 3.5
<210> 1
   <211> 959
   <212> PRT
   <213> Streptococcus pyogenes
<400> 1
   Glu Glu Lys Thr Val Gln Val Gln Lys Gly Leu Pro Ser Ile Asp Ser
   1               5                   10                  15
   Leu His Tyr Leu Ser Glu Asn Ser Lys Lys Glu Phe Lys Glu Glu Leu
               20                  25                  30
   Ser Lys Ala Gly Gln Glu Ser Gln Lys Val Lys Glu Ile Leu Ala Lys
           35                  40                  45
   Ala Gln Gln Ala Asp Lys Gln Ala Gln Glu Leu Ala Lys Met Lys Ile
       50                  55                  60
   Pro Glu Lys Ile Pro Met Lys Pro Leu His Gly Pro Leu Tyr Gly Gly
   65                  70                  75                  80
   Tyr Phe Arg Thr Trp His Asp Lys Thr Ser Asp Pro Thr Glu Lys Asp
                   85                  90                  95
   Lys Val Asn Ser Met Gly Glu Leu Pro Lys Glu Val Asp Leu Ala Phe
               100                 105                 110
   Ile Phe His Asp Trp Thr Lys Asp Tyr Ser Leu Phe Trp Lys Glu Leu
           115                 120                 125
   Ala Thr Lys His Val Pro Lys Leu Asn Lys Gln Gly Thr Arg Val Ile
       130                 135                 140
   Arg Thr Ile Pro Trp Arg Phe Leu Ala Gly Gly Asp Asn Ser Gly Ile
   145                 150                 155                 160
   Ala Glu Asp Thr Ser Lys Tyr Pro Asn Thr Pro Glu Gly Asn Lys Ala
                   165                 170                 175
   Leu Ala Lys Ala Ile Val Asp Glu Tyr Val Tyr Lys Tyr Asn Leu Asp
   180 185 190
   Gly Leu Asp Val Asp Val Glu His Asp Ser Ile Pro Lys Val Asp Lys
   195 200 205
   Lys Glu Asp Thr Ala Gly Val Glu Arg Ser Ile Gln Val Phe Glu Glu
   210 215 220
   Ile Gly Lys Leu Ile Gly Pro Lys Gly Val Asp Lys Ser Arg Leu Phe
   225 230 235 240
   Ile Met Asp Ser Thr Tyr Met Ala Asp Lys Asn Pro Leu Ile Glu Arg
   245 250 255
   Gly Ala Pro Tyr Ile Asn Leu Leu Leu Val Gln Val Tyr Gly Ser Gln
   260 265 270
   Gly Glu Lys Gly Gly Trp Glu Pro Val Ser Asn Arg Pro Glu Lys Thr
   275 280 285
   Met Glu Glu Arg Trp Gln Gly Tyr Ser Lys Tyr Ile Arg Pro Glu Gln
   290 295 300
   Tyr Met Ile Gly Phe Ser Phe Tyr Glu Glu Asn Ala Gln Glu Gly Asn
   305 310 315 320
   Leu Trp Tyr Asp Ile Asn Ser Arg Lys Asp Glu Asp Lys Ala Asn Gly
   325 330 335
   Ile Asn Thr Asp Ile Thr Gly Thr Arg Ala Glu Arg Tyr Ala Arg Trp
   340 345 350
   Gln Pro Lys Thr Gly Gly Val Lys Gly Gly Ile Phe Ser Tyr Ala Ile
   355 360 365
   Asp Arg Asp Gly Val Ala His Gln Pro Lys Lys Tyr Ala Lys Gln Lys
   370 375 380
   Glu Phe Lys Asp Ala Thr Asp Asn Ile Phe His Ser Asp Tyr Ser Val
   385 390 395 400
   Ser Lys Ala Leu Lys Thr Val Met Leu Lys Asp Lys Ser Tyr Asp Leu
   405 410 415
   Ile Asp Glu Lys Asp Phe Pro Asp Lys Ala Leu Arg Glu Ala Val Met
   420 425 430
   Ala Gln Val Gly Thr Arg Lys Gly Asp Leu Glu Arg Phe Asn Gly Thr
           435                 440                 445
   Leu Arg Leu Asp Asn Pro Ala Ile Gln Ser Leu Glu Gly Leu Asn Lys
       450                 455                 460
   Phe Lys Lys Leu Ala Gln Leu Asp Leu Ile Gly Leu Ser Arg Ile Thr
   465                 470                 475                 480
   Lys Leu Asp Arg Ser Val Leu Pro Ala Asn Met Lys Pro Gly Lys Asp
                   485                 490                 495
   Thr Leu Glu Thr Val Leu Glu Thr Tyr Lys Lys Asp Asn Lys Glu Glu
               500                 505                 510
   Pro Ala Thr Ile Pro Pro Val Ser Leu Lys Val Ser Gly Leu Thr Gly
           515                 520                 525
   Leu Lys Glu Leu Asp Leu Ser Gly Phe Asp Arg Glu Thr Leu Ala Gly
       530                 535                 540
   Leu Asp Ala Ala Thr Leu Thr Ser Leu Glu Lys Val Asp Ile Ser Gly
   545                 550                 555                 560
   Asn Lys Leu Asp Leu Ala Pro Gly Thr Glu Asn Arg Gln Ile Phe Asp
                   565                 570                 575
   Thr Met Leu Ser Thr Ile Ser Asn His Val Gly Ser Asn Glu Gln Thr
               580                 585                 590
   Val Lys Phe Asp Lys Gln Lys Pro Thr Gly His Tyr Pro Asp Thr Tyr
           595                 600                 605
   Gly Lys Thr Ser Leu Arg Leu Pro Val Ala Asn Glu Lys Val Asp Leu
       610                 615                 620
   Gln Ser Gln Leu Leu Phe Gly Thr Val Thr Asn Gln Gly Thr Leu Ile
   625                 630                 635                 640
   Asn Ser Glu Ala Asp Tyr Lys Ala Tyr Gln Asn His Lys Ile Ala Gly
                   645                 650                 655
   Arg Ser Phe Val Asp Ser Asn Tyr His Tyr Asn Asn Phe Lys Val Ser
               660                 665                 670
   Tyr Glu Asn Tyr Thr Val Lys Val Thr Asp Ser Thr Leu Gly Thr Thr
               675                 680                 685
   Thr Asp Lys Thr Leu Ala Thr Asp Lys Glu Glu Thr Tyr Lys Val Asp
           690                 695                 700
   Phe Phe Ser Pro Ala Asp Lys Thr Lys Ala Val His Thr Ala Lys Val
   705                 710                 715                 720
   Ile Val Gly Asp Glu Lys Thr Met Met Val Asn Leu Ala Glu Gly Ala
                   725                 730                 735
   Thr Val Ile Gly Gly Ser Ala Asp Pro Val Asn Ala Arg Lys Val Phe
               740                 745                 750
   Asp Gly Gln Leu Gly Ser Glu Thr Asp Asn Ile Ser Leu Gly Trp Asp
           755                 760                 765
   Ser Lys Gln Ser Ile Ile Phe Lys Leu Lys Glu Asp Gly Leu Ile Lys
       770                 775                 780
   His Trp Arg Phe Phe Asn Asp Ser Ala Arg Asn Pro Glu Thr Thr Asn
   785                 790                 795                 800
   Lys Pro Ile Gln Glu Ala Ser Leu Gln Ile Phe Asn Ile Lys Asp Tyr
                   805                 810                 815
   Asn Leu Asp Asn Leu Leu Glu Asn Pro Asn Lys Phe Asp Asp Glu Lys
               820                 825                 830
   Tyr Trp Ile Thr Val Asp Thr Tyr Ser Ala Gln Gly Glu Arg Ala Thr
           835                 840                 845
   Ala Phe Ser Asn Thr Leu Asn Asn Ile Thr Ser Lys Tyr Trp Arg Val
       850                 855                 860
   Val Phe Asp Thr Lys Gly Asp Arg Tyr Ser Ser Pro Val Val Pro Glu
   865                 870                 875                 880
   Leu Gln Ile Leu Gly Tyr Pro Leu Pro Asn Ala Asp Thr Ile Met Lys
                   885                 890                 895
   Thr Val Thr Thr Ala Lys Glu Leu Ser Gln Gln Lys Asp Lys Phe Ser
               900                 905                 910
   Gln Lys Met Leu Asp Glu Leu Lys Ile Lys Glu Met Ala Leu Glu Thr
           915                 920                 925
   Ser Leu Asn Ser Lys Ile Phe Asp Val Thr Ala Ile Asn Ala Asn Ala
   930 935 940
   Gly Val Leu Lys Asp Cys Ile Glu Lys Arg Gln Leu Leu Lys Lys
   945 950 955
<210> 2
   <211> 995
   <212> PRT
   <213> Streptococcus pyogenes
<400> 2
   Met Asp Lys His Leu Leu Val Lys Arg Thr Leu Gly Cys Val Cys Ala
   1 5 10 15
   Ala Thr Leu Met Gly Ala Ala Leu Ala Thr His His Asp Ser Leu Asn
   20 25 30
   Thr Val Lys Ala Glu Glu Lys Thr Val Gln Val Gln Lys Gly Leu Pro
   35 40 45
   Ser Ile Asp Ser Leu His Tyr Leu Ser Glu Asn Ser Lys Lys Glu Phe
   50 55 60
   Lys Glu Glu Leu Ser Lys Ala Gly Gln Glu Ser Gln Lys Val Lys Glu
   65 70 75 80
   Ile Leu Ala Lys Ala Gln Gln Ala Asp Lys Gln Ala Gln Glu Leu Ala
   85 90 95
   Lys Met Lys Ile Pro Glu Lys Ile Pro Met Lys Pro Leu His Gly Pro
   100 105 110
   Leu Tyr Gly Gly Tyr Phe Arg Thr Trp His Asp Lys Thr Ser Asp Pro
   115 120 125
   Thr Glu Lys Asp Lys Val Asn Ser Met Gly Glu Leu Pro Lys Glu Val
   130 135 140
   Asp Leu Ala Phe Ile Phe His Asp Trp Thr Lys Asp Tyr Ser Leu Phe
   145 150 155 160
   Trp Lys Glu Leu Ala Thr Lys His Val Pro Lys Leu Asn Lys Gln Gly
   165 170 175
   Thr Arg Val Ile Arg Thr Ile Pro Trp Arg Phe Leu Ala Gly Gly Asp
   180 185 190
   Asn Ser Gly Ile Ala Glu Asp Thr Ser Lys Tyr Pro Asn Thr Pro Glu
           195                 200                 205
   Gly Asn Lys Ala Leu Ala Lys Ala Ile Val Asp Glu Tyr Val Tyr Lys
       210                 215                 220
   Tyr Asn Leu Asp Gly Leu Asp Val Asp Val Glu His Asp Ser Ile Pro
   225                 230                 235                 240
   Lys Val Asp Lys Lys Glu Asp Thr Ala Gly Val Glu Arg Ser Ile Gln
                   245                 250                 255
   Val Phe Glu Glu Ile Gly Lys Leu Ile Gly Pro Lys Gly Val Asp Lys
               260                 265                 270
   Ser Arg Leu Phe Ile Met Asp Ser Thr Tyr Met Ala Asp Lys Asn Pro
           275                 280                 285
   Leu Ile Glu Arg Gly Ala Pro Tyr Ile Asn Leu Leu Leu Val Gln Val
       290                 295                 300
   Tyr Gly Ser Gln Gly Glu Lys Gly Gly Trp Glu Pro Val Ser Asn Arg
   305                 310                 315                 320
   Pro Glu Lys Thr Met Glu Glu Arg Trp Gln Gly Tyr Ser Lys Tyr Ile
                   325                 330                 335
   Arg Pro Glu Gln Tyr Met Ile Gly Phe Ser Phe Tyr Glu Glu Asn Ala
               340                 345                 350
   Gln Glu Gly Asn Leu Trp Tyr Asp Ile Asn Ser Arg Lys Asp Glu Asp
           355                 360                 365
   Lys Ala Asn Gly Ile Asn Thr Asp Ile Thr Gly Thr Arg Ala Glu Arg
       370                 375                 380
   Tyr Ala Arg Trp Gln Pro Lys Thr Gly Gly Val Lys Gly Gly Ile Phe
   385                 390                 395                 400
   Ser Tyr Ala Ile Asp Arg Asp Gly Val Ala His Gln Pro Lys Lys Tyr
                   405                 410                 415
   Ala Lys Gln Lys Glu Phe Lys Asp Ala Thr Asp Asn Ile Phe His Ser
               420                 425                 430
   Asp Tyr Ser Val Ser Lys Ala Leu Lys Thr Val Met Leu Lys Asp Lys
           435                 440                 445
   Ser Tyr Asp Leu Ile Asp Glu Lys Asp Phe Pro Asp Lys Ala Leu Arg
       450                 455                 460
   Glu Ala Val Met Ala Gln Val Gly Thr Arg Lys Gly Asp Leu Glu Arg
   465                 470                 475                 480
   Phe Asn Gly Thr Leu Arg Leu Asp Asn Pro Ala Ile Gln Ser Leu Glu
                   485                 490                 495
   Gly Leu Asn Lys Phe Lys Lys Leu Ala Gln Leu Asp Leu Ile Gly Leu
               500                 505                 510
   Ser Arg Ile Thr Lys Leu Asp Arg Ser Val Leu Pro Ala Asn Met Lys
           515                 520                 525
   Pro Gly Lys Asp Thr Leu Glu Thr Val Leu Glu Thr Tyr Lys Lys Asp
       530                 535                 540
   Asn Lys Glu Glu Pro Ala Thr Ile Pro Pro Val Ser Leu Lys Val Ser
   545                 550                 555                 560
   Gly Leu Thr Gly Leu Lys Glu Leu Asp Leu Ser Gly Phe Asp Arg Glu
                   565                 570                 575
   Thr Leu Ala Gly Leu Asp Ala Ala Thr Leu Thr Ser Leu Glu Lys Val
               580                 585                 590
   Asp Ile Ser Gly Asn Lys Leu Asp Leu Ala Pro Gly Thr Glu Asn Arg
           595                 600                 605
   Gln Ile Phe Asp Thr Met Leu Ser Thr Ile Ser Asn His Val Gly Ser
       610                 615                 620
   Asn Glu Gln Thr Val Lys Phe Asp Lys Gln Lys Pro Thr Gly His Tyr
   625                 630                 635                 640
   Pro Asp Thr Tyr Gly Lys Thr Ser Leu Arg Leu Pro Val Ala Asn Glu
                   645                 650                 655
   Lys Val Asp Leu Gln Ser Gln Leu Leu Phe Gly Thr Val Thr Asn Gln
               660                 665                 670
   Gly Thr Leu Ile Asn Ser Glu Ala Asp Tyr Lys Ala Tyr Gln Asn His
           675                 680                 685
   Lys Ile Ala Gly Arg Ser Phe Val Asp Ser Asn Tyr His Tyr Asn Asn
       690                 695                 700
   Phe Lys Val Ser Tyr Glu Asn Tyr Thr Val Lys Val Thr Asp Ser Thr
   705 710 715 720
   Leu Gly Thr Thr Thr Asp Lys Thr Leu Ala Thr Asp Lys Glu Glu Thr
   725 730 735
   Tyr Lys Val Asp Phe Phe Ser Pro Ala Asp Lys Thr Lys Ala Val His
   740 745 750
   Thr Ala Lys Val Ile Val Gly Asp Glu Lys Thr Met Met Val Asn Leu
   755 760 765
   Ala Glu Gly Ala Thr Val Ile Gly Gly Ser Ala Asp Pro Val Asn Ala
   770 775 780
   Arg Lys Val Phe Asp Gly Gln Leu Gly Ser Glu Thr Asp Asn Ile Ser
   785 790 795 800
   Leu Gly Trp Asp Ser Lys Gln Ser Ile Ile Phe Lys Leu Lys Glu Asp
   805 810 815
   Gly Leu Ile Lys His Trp Arg Phe Phe Asn Asp Ser Ala Arg Asn Pro
   820 825 830
   Glu Thr Thr Asn Lys Pro Ile Gln Glu Ala Ser Leu Gln Ile Phe Asn
   835 840 845
   Ile Lys Asp Tyr Asn Leu Asp Asn Leu Leu Glu Asn Pro Asn Lys Phe
   850 855 860
   Asp Asp Glu Lys Tyr Trp Ile Thr Val Asp Thr Tyr Ser Ala Gln Gly
   865 870 875 880
   Glu Arg Ala Thr Ala Phe Ser Asn Thr Leu Asn Asn Ile Thr Ser Lys
   885 890 895
   Tyr Trp Arg Val Val Phe Asp Thr Lys Gly Asp Arg Tyr Ser Ser Pro
   900 905 910
   Val Val Pro Glu Leu Gln Ile Leu Gly Tyr Pro Leu Pro Asn Ala Asp
   915 920 925
   Thr Ile Met Lys Thr Val Thr Thr Ala Lys Glu Leu Ser Gln Gln Lys
   930 935 940
   Asp Lys Phe Ser Gln Lys Met Leu Asp Glu Leu Lys Ile Lys Glu Met
   945                 950                 955                 960
   Ala Leu Glu Thr Ser Leu Asn Ser Lys Ile Phe Asp Val Thr Ala Ile
                   965                 970                 975
   Asn Ala Asn Ala Gly Val Leu Lys Asp Cys Ile Glu Lys Arg Gln Leu
               980                 985                 990
   Leu Lys Lys
           995

## Description

The invention relates to compositions for use in methods of treating disease, wherein the methods increase the potency of therapeutic antibodies.

Historically, the original aim of using antibodies, particularly monoclonal antibodies, for the treatment of diseases such as cancer was as 'magic bullets' to seek and destroy cancer cells in the body.

Antibodies can be used to recruit the immune system to particular targets within the body. Antibodies recruit the cellular immune system through the interaction of the antibody fragment crystallisable (Fc) domain with Fc receptors (FcRs) expressed on the surface of immune cells. The interaction between the Fc domain of a therapeutic antibody and an FcR is important in a range of antibody therapeutics in development including: anti-cancer antibodies, anti-inflammatory antibodies, anti-pathogen antibodies and antibodies for the treatment of autoimmunity.

In such therapies, antibodies are specific to target antigens through the specificity of the Fab domains. These antibodies are usually of the immunoglobulin G (IgG) class: IgG1, IgG2, IgG3 and IgG4. These antibodies bind the human FcRs: FcγRI, RγIIa, RγIIb, RγIIIa and FcγRn, and the complement Fc receptor C1q. The efficacy of the recruitment of the cellular immune system by IgG molecules is influenced by the affinity of the Fc to the FcR(s). Thus, variants of IgG Fc have been developed that exhibit modulated binding to FcRs. These IgG Fc variants have changes introduced to the protein and/or carbohydrate component of the Fc domain to change the affinity to one or more FcR and they may exhibit decreased or increased affinity to one or more FcR. This modulation in receptor affinity enables the recruitment of a particular range of cellular and humoral immune components with a particular pro-inflammatory or anti-inflammatory profiles. Many antigens have been discovered that are selectively or uniquely expressed on particular cancers, but generally attempts to use unmodified antibodies to kill cancer cells have met with only moderate success. The problem in using unlabelled 'plain' antibodies has been that cancer cells, like other host cells, are protected against complement mediated lysis, by expression of inhibitors of complement fixation (such as
decay accelerating factor, DAF) on the cell surface. Likewise, the concentration or abundance of cancer antigens at the surface of cells is sometimes very low, and insufficient to support killing by antibody directed effector mechanisms such as ADCC and complement fixation.

As a result of these problems of low cancer antigen abundance on the surface of cells, monoclonal antibody products have been developed for killing cancer cells that recognize tissue or lineage-specific antigens that are abundant on the cancer cells, but also present on host cells, which compromises the 'specificity' advantage of cancer specific antibodies directed at cancer-specific antigens. Examples include Rituxan (anti CD20) for non-Hodgkin's lymphoma (CD20 is also expressed on normal B-cells), likewise Yermoy for metastatic melanoma (which recognizes CTLA4, also expressed on normal T-cells).

Because of these problems encountered in developing the original 'magic bullet' concept (of unlabelled antibodies capable of selectively killing only cancer cells) further strategies have been developed exploiting the specificity attainable with monoclonal antibodies for growth factors (e.g. VEGF, which is recognized by Avastin) or growth factor receptors (eg. Her2, which is recognized by Herceptin) which are important for the growth of cancer cells. In the case of VEGF however, it serves important physiological functions which are also neutralized by Avastin, defeating to some extent the theoretical advantage of antibodies that would recognize cancer specific antigens and kill the cells bearing them, without killing healthy cells.

In addition to the treatment of cancer, monoclonal antibodies can be used for the treatment of viral infections whereby aberrantly presented or elevated host cell antigens and/or virally encoded antigens are targeted by the monoclonal. Elimination of the pathogen occurs through similar FcR dependent mechanisms. For example bavituximab targets the aberrant presentation of phosphatidyserine in the outer leaflet of the plasma membrane of infected cells.

A number of other approaches have been adopted to enhance the properties of antibodies.

One approach is to directly cross-link an antigen with an FcR using a bispecific antibody. Similar approaches have been described using Fc-free formats: for example multiply-presented Fv fragments which bring together two target proteins.

As mentioned above, an increasingly common approach to increasing cytotoxic activity is to conjugate toxins directly the Fc or to the antibody-like molecule.

One method for the enhancement of IgG Fc binding to FcγRs has been the manufacture of IgG with mutated Fc regions. Mutations, notably in the tip of the Cγ2 domains that form the receptor binding surface or those proximal to the receptor binding surface, can lead to increased FcR interactions. For example, Shields *et al.,* introduced mutations into the Fc and generated altered Fc receptor binding (Shields et al, J Biol Chem. 2001 Mar 2;276(9):6591-604).

A further method for the enhancement of IgG Fc binding to FcγRIIIa has been the manufacture of IgG Fc lacking alpha1-6 fucose on the Asn297-linked *N*-acetyl glucosamine (GlcNAc) (so-called "core" fucose). The presence of fucose in that position decreases the affinity with FcγRIIIa by clashing with the FcγRIIIa glycan at Asn162. Removal of the fucose eliminates this steric clash and increases the affinity to FcyRIIIa.

Whilst engineering antibodies to exhibit enhanced FcR binding achieves some improvement in the recruitment of cellular immune cells, these methods are intrinsically limited by the availability of the unbound receptors. At physiological conditions, the majority of activating cellular FcγRs are bound to Fc as a result of a serum antibody concentration in significant excess of the dissociation constant for Fc:FcR interactions (Preithner, S., Elm, S., Lippold, S., Locher, M., Wolf, A., da Silva, A. J., Baeuerle, P. A. & Prang, N. S. (2006). Mol Immunol 43, 1183-93; Bruhns, P., Iannascoli, B., England, P., Mancardi, D. A., Fernandez, N., Jorieux, S. & Daeron, M. (2009). Blood 113, 3716-25.). Activation of cellular FcγRs is therefore dependent upon the displacement of irrelevant antibody (bulk serum IgG) prior to the binding of IgG presented by immune complexes or polyvalent antigens.

Engineering of either or both of the carbohydrate or protein component of the Fc can help overcome the competitive effect of serum IgG for antigens expressed at high levels on the cell surface, low affinity or low-copy epitopes on infected or cancerous cells are largely protected from antibody-dependent cellular cytotoxicity (ADCC) by serum IgG.

Nandakumar et al (2008) Trends in Immunology 29:4 p173-178 relates to therapeutic cleavage of IgG and new avenues for treating inflammation.

WO2008/071418 A2 relates to the use of an EndoS polypeptide.

WO2009/033670 A2 relates to methods and kits for the dissociation of Fcγ-receptor-IgG complexes and methods and kits for the isolation of IgG and Fc and Fab fragments of IgG.

There is still a need, however, to develop new methods for enhancing or favouring the interaction between the Fc domain of therapeutic antibodies and FcγRs.

Whilst enzymes currently exist which are capable of modifying IgG and reducing its binding to FcR, these enzymes have the disadvantage that they lead to the decreased binding of all antibodies to FcγRs (both bulk serum antibodies and, additionally, any introduced therapeutic antibodies if also present in the serum). Therefore, while these enzymes may be useful in cases where serum antibody is itself responsible for pathology they do not, by themselves enhance or favour the interaction between introduced therapeutic antibodies and FcγRs (because such therapeutic antibodies would be affected by the enzyme in the same way as serum IgG). Thus, to date, these IgG-modifying-enzymes have only been used therapeutically for the treatment of autoimmune disorders driven by endogenous serum auto-antibody (Albert H, Collin M, Dudziak D, Ravetch JV, Nimmerjahn F. Proc Natl Acad Sci U S A. 2008 Sep 30;105(39):15005-9.).

A new approach have now been developed to enhancing the killing power of antibodies that can be applied to tumour-specific antigens that are selectively expressed on cancer cells and other cells. It can also be applied to antibody strategies to neutralize essential growth factors by enhancing the Fc-receptor mediated clearance of said growth factors.

In particular, the inventors have now recognized that, in order to achieve the selective enzymatic removal of serum IgG but not any co-administered therapeutic antibody, the two antibody populations would need to exhibit differential sensitivities to the enzyme or the enzyme and therapeutic antibodies would have to be kept spatially or temporally apart.

The present invention shows how differential sensitivity to IgG modifying-agents, and enzymes in particular, may be achieved to allow the removal of serum antibodies but not engineered IgG.

It is demonstrated that the binding of bulk serum IgG to FcRs can be reduced whilst at the same time enhancing the functional binding to FcRs of a therapeutic antibody which may be exogenously introduced. This control can be achieved by the introduction of an agent which is capable of reducing or eliminating the FcR binding properties of serum IgG, together with a therapeutic antibody that is resistant to such activity of the agent. Specifically, this applies to the administration of an Fc degrading agent with a therapeutic antibody with an Fc engineered or chosen to be resistant to the Fc-degrading agent.

It is also demonstrated that inhibition of FcR binding by agents that reduce or eliminate serum Fc binding will enhance the FcR binding of a therapeutic antibody resistant to such an agent. In particular, it is shown herein that the deglycosylation of competing serum IgG has a relatively modest effect on the apparent affinity of serum IgG for FcgR. This would be expected to correspond to an equivalently modest increase in affinity for an unaffected antibody. However, the inventors' data shows that an approximately 10-fold decrease in serum-FcγR affinity leads surprisingly to an approximately 1000-fold increase in monoclonal antibody efficacy. It is believed that such an enhancement in FcR binding will lead to enhanced recruitment of cellular immune functions targeted by the therapeutic antibodies. These cellular immune functions may be either pro-inflammatory or anti-inflammatory depending on the particular Fc variant.

Accordingly, the present invention provides an agent which reduces Fc receptor binding by enzymatic degradation of endogenous serum antibodies for use in a method of treating a disease, wherein the agent is an endoglycosidase, a protease or a protein-N-glyconase and the method increases the therapeutic potency of an antibody, wherein the mode of action of said antibody relies on the Fc:FcR interaction, and wherein the method comprises the steps:
(a) administering said agent to a subject; and subsequently,
(b) after a set time interval, administering said antibody to the subject.

Disclosed herein is a composition comprising a therapeutically effective amount of the agent and the antibody.

Also disclosed herein is a pharmaceutical composition further comprising a pharmaceutically acceptable carrier or diluent.

Preferably, the (i) agent which reduces Fc receptor binding by enzymatic degradation of endogenous serum antibodies and (ii) the antibody, preferably which is resistant to the agent, are for use in a method of treating cancer, infection and/or autoimmunity. Preferably, the (i) agent which reduces Fc receptor binding by enzymatic degradation of endogenous serum antibodies and (ii) the antibody, preferably which is resistant to the agent, are for use in a method of treating cancer.

The invention enables lower doses of the therapeutic antibody to be used and/or side effects of the therapeutic antibody to be reduced.

The set time interval provides time for the agent to act on the endogenous serum antibodies within the subject to reduce Fc receptor binding. Preferably, the amount of agent administered and the time interval is sufficient to reduce the Fc receptor binding by endogenous serum antibodies in the subject to below 50% of the starting levels of Fc receptor binding (i.e. to below 50% of the levels of Fc receptor binding by endogenous serum antibodies before treatment with the agent). More preferably, the amount of agent administered and the time interval is sufficient to reduce the Fc receptor binding levels of serum antibodies in the subject to below 40%, 30%, 20% or 10% of the starting levels in that patient. The agent may be administered at one single time point or over a set time.

The time interval might, for example, be 1-2, 1-5, 1-10 or 1-20 days. The amount of agent might be 0.1 mg/Kg, 1 mg/Kg or 10 mg/Kg. For example 1-50mg EndoS may be used, preferably 10-20mg EndoS, more preferably about 20mg EndoS. Endo S has a half life of less than 12 hours *in vivo.* In cases where the agent is EndoS, the time interval may therefore be 1-5, more preferably 3-4 days.

In yet other embodiments, the method comprising the steps:
(a) treating blood from the subject *ex vivo* with the agent;
(b) returning the blood to the subject; and subsequently
(c) administering said antibody to the subject.

The agent that reduces Fc receptor binding of endogenous serum antibodies is an agent that interrupts Fc:FcR interaction by enzymatic degradation of serum antibodies.

The agent is a protease, an endoglycosidase or a protein N-glycanase. Preferably, the agent cleaves serum antibodies in the Fc region preventing or reducing Fc receptor binding.

Preferably the agent is an endoglycosidase. Preferably the endoglycosidase is specific for the types of glycans found on the Fc of natural serum IgG. Preferably the endoglycosidase is endoglycosidase S, F3 or Ebeta. Preferably the endoglycosidase is endoglycosidase S (EndoS). Preferably, the endoglycosidase is endoglycosidase F3. Preferably, the endoglycosidase is endoglycosidase Ebeta.

In a preferred embodiment, the agent is the endoglycosidase, EndoS. The inventors have surprisingly found that EndoS is able to enhance FcγR binding and may thereby act as an immunoactivatory agent to selectively eliminate FcγR binding to bulk serum antibody and not FcγR binding to a therapeutic antibody of interest which is engineered so as to be resistant to EndoS.

The agent that reduces Fc receptor binding by enzymatic degradation of endogenous serum antibodies may be a protease. Preferably the protease is IdeS.

The therapeutic antibody may be a human monoclonal antibody, or recombinant antibody, or one or more fragments thereof. If the antibody is a recombinant antibody it may be humanised. Alternatively, the therapeutic antibody may be a polyclonal antibody. The therapeutic antibody must be one which comprises an Fc domain.

The Fc domain of the therapeutic antibody resistant to activity of the agent may comprise one or more glycoforms resistant to the activity of the agent. The glycoform may be an oligomannose-type glycoform. Preferably, the oligomannose-type glycoform comprises Man₅GlcNAc₂, Man₈GlcNAc₂ or Man₉GlcNAc₂. in the Fc domain. Preferably the oligomannose-type glycoform is a mixture of oligomannose-type glycans. Preferably, the oligomannose-type glycoform is an oligomannose-type glycan or 'yeast-type' oligomannose-type glycan which contains between 5 and 20 mannose residues in each of the glycans of the Fc domain. More preferably, the oligomannose-type glycan or the yeast-type oligomannose-type glycan contains 5 mannoses, or 6 mannoses, or 7 mannoses, or 8 mannose, or 9 mannoses, or 10 mannose, or 11 mannoses, or 12 mannoses, or 13 mannoses, or 14 mannoses, or 15 mannoses, or 16 mannoses, or 17 mannoses, or 18 mannoses, or 19 mannoses or 20 mannoses in each of the glycans of the Fc domain. Preferably, the oligomannose-type glycan or the yeast-type oligomannose-type glycan contains at least 5 mannose residues in each of the glycans of the Fc domain. More preferably, the oligomannose-type glycan or the yeast-type oligomannose-type glycan contains no more than 15 mannose residues in each of the glycans of the Fc domain. Preferably, the oligomannose-type glycan or 'yeast-type' oligomannose-type glycan contains only two GlcNAc residues and three or more mannose residues in each of the glycans of the Fc domain.

The glycoform may alternatively contain a 'bisecting-*N*-acetylglucosamine' (bisecting GlcNAc). Preferably, the bisecting-*N*-acetylglucosamine is *N-*acetylglucosamine b1-4 linked to a beta-mannose residue. More preferably, the glycoform contains at least one beta -*N*-acetylglucosamine residue 1-4 linked to a beta-mannose residue in each of the glycans of the Fc domain. Preferably, the glycoform contains two beta-*N*-acetylglucosamine residues 1-4 linked to a beta-mannose residue in each of the glycans of the Fc domain.

The glycoform may contain sialic acid. More preferably, the glycoform contains sialic acid alpha 2-6-linked to galactose. Preferably, the glycoform contains one or two sialic acid residues attached to each of the two glycans of the Fc domain.

The glycoform may be a hybrid-type. Preferably, the hybrid-type is Man₅GlcNAc₂ modified with additional complex-type residues on the 3-arm of the trimannosyl core. Preferably, the hybrid-type glycoform contains *N*-acetylglucosamine b1-4 linked to a beta-mannose residue (bisecting GlcNAc).

In an alternative embodiment the antibody may be engineered to be aglycosylated, that is, there are no glycan groups on the Fc domains, whilst still competent for Fc receptor binding as described in (Sazinsky et al. PNAS 2008 Dec 23: 105(51):20167-20172).

Preferably, a therapeutic antibody resistant to the endoglycosidase activity of the agent comprises one or more glycoforms resistant to the endoglycosidase activity of the agent. The glycoform may be an oligomannose-type glycoform. Preferably, the oligomannose-type glycoform comprises Man₅GlcNAc₂, Man₈GlcNAc₂, or Man₉GlcNAc₂ in the Fc domain. The glycoform may alternatively be a 'bisecting - *N-*acetylglucosamine'. The glycoform may contain sialic acid. The glycoform may contain sialic acid alpha 2-6-linked to galactose.

A therapeutic antibody resistant to the endoglycosidase activity of EndoS may comprise one or more glycoforms resistant to the endoglycosidase activity of EndoS. Preferably, the glycoform is an oligomannose-type glycoform. Preferably, the oligomannose-type glycoform comprises Man₅GlcNAc₂, Man₈GlcNAc₂ or Man₉GlcNAc₂ in the Fc domain. The glycoform may alternatively contain a 'bisecting - *N*-acetylglucosamine.' The glycoform may contain sialic acid. The glycoform may contain sialic acid alpha 2-6-linked to galactose.

Preferably, a therapeutic antibody resistant to the endoglycosidase activity of EndoF3 comprises one or more glycoforms resistant to the endoglycosidase activity of EndoF3. Preferably, the glycoform is an oligomannose-type glycoform. Preferably, the oligomannose-type glycoform is Man₅GlcNAc₂, Man₈GlcNAc₂, or Man₉GlcNAc₂. The glycoform may alternatively be a 'bisecting - *N*-acetylglucosamine.' The glycoform may contain sialic acid. The glycoform may contain sialic acid alpha 2-6-linked to galactose.

In other embodiments of the invention, the therapeutic antibody is one which is not resistant to the agent as defined herein, e.g. the therapeutic antibody is not resistant to (i.e. it is sensitive to) an agent (e.g. EndoS or IdeS) which reduces Fc receptor binding of endogenous serum antibodies.

In a preferred embodiment, EndoS which reduces Fc receptor binding of endogenous serum antibodies and a therapeutic antibody, preferably a therapeutic antibody which is resistant to the endoglycosidase activity of EndoS, are for use in treating cancer.

In another preferred embodiment, EndoS which reduces Fc receptor binding of endogenous serum antibodies and a glycoform of a therapeutic antibody resistant to the endoglycosidase activity of EndoS are for use in treating cancer, infection or and/or autoimmunity. Preferably, EndoS which reduces Fc receptor binding of endogenous serum antibodies and a glycoform of an antibody resistant to the endoglycosidase activity of EndoS are for use in treating cancer.

Disclosed herein is a product containing EndoS which reduces Fc receptor binding of endogenous serum antibodies and a therapeutic antibody, preferably a therapeutic antibody which is resistant to the endoglycosidase activity of EndoS, as a combined preparation for simultaneous, separate or sequential use in a method of treating cancer, infection and/or autoimmunity. Preferably, the combined preparation is for use in a method of treating cancer.

Also disclosed herein is a product containing EndoS which reduces Fc receptor binding of endogenous serum antibodies and a glycoform of a therapeutic antibody resistant to endoglycosidase activity of EndoS as a combined preparation for simultaneous, separate or sequential use in a method of treating cancer, infection and/or autoimmunity. Preferably, the combined preparation is for use in treating cancer.

In some embodiments, the cancer is selected from the group consisting of Acute lymphoblastic leukemia, Acute myeloid leukemia, Adrenocortical carcinoma, AIDS-related cancers, AIDS-related lymphoma, Anal cancer, Appendix cancer, Astrocytoma, childhood cerebellar or cerebral, Basal cell carcinoma, Bile duct cancer, extrahepatic, Bladder cancer, Bone cancer, Osteosarcoma/Malignant fibrous histiocytoma, Brainstem glioma, Brain cancer, Brain tumor, cerebellar astrocytoma, Brain tumor, cerebral astrocytoma/malignant glioma, Brain tumor, ependymoma, Brain tumor, medulloblastoma, Brain tumor, supratentorial primitive neuroectodermal tumors, Brain tumor, visual pathway and hypothalamic glioma, Breast cancer, Bronchial adenomas/carcinoids, Burkitt lymphoma, Carcinoid tumor, Carcinoid tumor, gastrointestinal, Carcinoma of unknown primary, Central nervous system lymphoma, Cerebellar astrocytoma, Cerebral astrocytoma/Malignant glioma, Cervical cancer, Chronic lymphocytic leukemia, Chronic myelogenous leukemia Chronic myeloproliferative disorders, Colon Cancer, Cutaneous T-cell lymphoma, Desmoplastic small round cell tumor, Endometrial cancer, Ependymoma, Esophageal cancer, Ewing's sarcoma in the Ewing family of tumors, Extracranial germ cell tumor, Childhood, Extragonadal Germ cell tumor, Extrahepatic bile duct cancer, Eye Cancer, Intraocular melanoma, Eye Cancer, Retinoblastoma, Gallbladder cancer, Gastric (Stomach) cancer, Gastrointestinal Carcinoid Tumor, Gastrointestinal stromal tumor (GIST), Germ cell tumor: extracranial, extragonadal, or ovarian, Gestational trophoblastic tumor, Glioma of the brain stem, Glioma, Childhood Cerebral Astrocytoma, Glioma, Childhood Visual Pathway and Hypothalamic, Gastric carcinoid, Hairy cell leukemia, Head and neck cancer, Heart cancer, Hepatocellular (liver) cancer, Hodgkin lymphoma, Hypopharyngeal cancer, Hypothalamic and visual pathway glioma, Intraocular Melanoma, Islet Cell Carcinoma (Endocrine Pancreas), Kaposi sarcoma, Kidney cancer (renal cell cancer), Laryngeal Cancer, Leukemias, Leukemia, acute lymphoblastic (also called acute lymphocytic leukemia), Leukemia, acute myeloid (also called acute myelogenous leukemia), Leukemia, chronic lymphocytic (also called chronic lymphocytic leukemia), Leukemia, chronic myelogenous (also called chronic myeloid leukemia), Leukemia, hairy cell, Lip and Oral Cavity Cancer, Liposarcoma, Liver Cancer (Primary), Lung Cancer, Non-Small Cell ,Lung Cancer, Small Cell, Lymphomas, Lymphoma, AIDS-related, Lymphoma, Burkitt, Lymphoma, cutaneous T-Cell, Lymphoma, Hodgkin, Lymphomas, Non-Hodgkin (an old classification of all lymphomas except Hodgkin's), Lymphoma, Primary Central Nervous System, Macroglobulinemia, Waldenstrom, Malignant Fibrous Histiocytoma of Bone/Osteosarcoma, Medulloblastoma, Melanoma, Melanoma, Intraocular (Eye), Merkel Cell Carcinoma, Mesothelioma, Adult Malignant, Mesothelioma, Metastatic Squamous Neck Cancer with Occult Primary, Mouth Cancer, Multiple Endocrine Neoplasia Syndrome, Multiple Myeloma/Plasma Cell Neoplasm, Mycosis Fungoides, Myelodysplastic Syndromes, Myelodysplastic/Myeloproliferative Diseases, Myelogenous Leukemia, Chronic, Myeloid Leukemia, Adult Acute, Myeloid Leukemia, Childhood Acute, Myeloma, Multiple (Cancer of the Bone-Marrow), Myeloproliferative Disorders, Nasal cavity and paranasal sinus cancer, Nasopharyngeal carcinoma, Neuroblastoma, Non-Hodgkin lymphoma, Non-small cell lung cancer, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, Pancreatic cancer, islet cell, Paranasal sinus and nasal cavity cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Plasma cell neoplasia/Multiple myeloma, Pleuropulmonary blastoma, Primary central nervous system lymphoma, Prostate cancer, Rectal cancer, Renal cell carcinoma (kidney cancer), Renal pelvis and ureter, transitional cell cancer, Retinoblastoma, Rhabdomyosarcoma, Salivary gland cancer, Sarcoma, Ewing family of tumors, Kaposi Sarcoma, Sarcoma, soft tissue, Sarcoma, uterine, Sézary syndrome, Skin cancer (nonmelanoma), Skin cancer (melanoma), Skin carcinoma, Merkel cell, Small cell lung cancer, Small intestine cancer, Soft tissue sarcoma, Squamous cell carcinoma, Squamous neck cancer with occult primary, metastatic, Stomach cancer, Supratentorial primitive neuroectodermal tumor, T-Cell lymphoma, cutaneous - see Mycosis Fungoides and Sézary syndrome, Testicular cancer, Throat cancer, Thymoma, Thymoma and Thymic carcinoma, Thyroid cancer, Thyroid cancer, Transitional cell cancer of the renal pelvis and ureter, Trophoblastic tumor, Ureter and renal pelvis, transitional cell cancer Urethral cancer, Uterine cancer, endometrial, Uterine sarcoma, Vaginal cancer, Visual pathway and hypothalamic glioma, Vulvar cancer, Waldenstrom macroglobulinemia and Wilms tumor (kidney cancer).

Preferably, the cancer is selected from the group consisting of bladder cancer, lung cancer, breast cancer, melanoma, colon cancer, rectal cancer, non-Hodgkin's lymphoma, endometrial cancer, pancreatic cancer, kidney (renal cell) cancer, prostate cancer, leukemia, oesophageal, or thyroid cancer, preferably breast cancer.

Also disclosed herein is a pharmaceutical composition for use in a method of treating cancer, infection and/or autoimmunity comprising:
(a) a therapeutically effective amount of EndoS which reduces Fc receptor binding of endogenous serum antibodies; and
(b) a therapeutically effective amount of a therapeutic antibody, preferably a therapeutic antibody which is resistant to the endoglycosidase activity of EndoS; and
(c) a pharmaceutically acceptable carrier or diluent.

Also disclosed herein is a pharmaceutical composition for use in a method of treating cancer, infection and/or autoimmunity comprising:
(a) a therapeutically effective amount of EndoS which reduces Fc receptor binding of endogenous serum antibodies; and
(b) a therapeutically effective amount of an oligomannose-type glycoform of a therapeutic antibody resistant to endoglycosidase activity of EndoS; and
(c) a pharmaceutically acceptable carrier or diluent.

As used herein, the term "subject" refers preferably to a mammal, most preferably to a human.

Unless otherwise defined herein, scientific and technical terms and phrases used in connection with the present invention shall have the meanings that are commonly understood by those of ordinary skill in the art. Further, unless otherwise required by context, singular terms shall include the plural and plural terms shall include the singular. Generally, nomenclatures used in connection with, and techniques of biochemistry, enzymology, molecular and cellular biology, microbiology, genetics and protein and nucleic acid chemistry and hybridization described herein are those well known and are commonly used in the art. The methods and techniques of the present invention are generally performed according to conventional methods well known in the art and as described in various general and more specific references that are cited and discussed throughout the present specification unless otherwise indicated. *See e.g.,* Sambrook et al. Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane, Antibodies: A Laboratory Manual, Cold Spring Harbor Laboratory Press, Cold Spring Harbor, N.Y. (1990); Taylor and Drickamer, Introduction to Glycobiology, Oxford Univ. Press (2003); Worthington Enzyme Manual, Worthington Biochemical Corp., Freehold, N.J.; Handbook of Biochemistry: Section A Proteins, Vol I, CRC Press (1976); .; Handbook of Biochemistry: Section A Proteins, Vol II, CRC Press (1976); Essentials of Glycobiology, Cold Spring Harbor Laboratory Press (1999); Immunobiology, Janeway et al., 6th Edition, 2004, Garland Publishing, New York).

The following terms, unless otherwise indicated, shall be understood to have the following meanings:
As used herein, 'an agent which reduces Fc receptor binding to endogenous serum antibodies' preferably refers to an agent which increases the equilibrium binding constant for the IgG:FcγR interaction by a factor of at least two. Preferably, the agent increases the equilibrium binding constant for the IgG:FcγR interaction by a factor of at least two, or at least 3, or at least 4, or at least 5, or at least 6, or at least 7 or at least 8. More preferably, the agent increases the equilibrium binding constant for the IgG:FcγR interaction by a factor of at least eight. An increase in the equilibrium binding constant represents a decrease in the binding between IgG and an FcγR (e.g. between IgG and FcγRIIA).

As used herein, the term 'endogenous serum antibodies' refers to gamma immunoglobulins (IgG1, IgG2, IgG3 and IgG4) which are present in human tissue which have been produced from an individual's B-cells or those gamma immunoglobulins which are already present prior to the administration of the current therapy. In some embodiments, the term 'endogenous serum antibodies' refers to glycosylated endogenous serum antibodies.

As used herein, the term 'FcγR' refers to Fc gamma immunoglobulin receptors which are present on human cells. In humans, FcγR refers to one, some, or all of the family of receptors comprising FcγRI (CD64), FcγRIIA (CD32), FcγRIIB (CD32), FcγRIIIA (CD16a) and FcγRIIIB (CD16b). As used herein, the term 'FcγR' includes naturally occurring polymorphisms of FcγRI (CD64), FcγRIIA (CD32), FcγRIIB (CD32), FcγRIIIA (CD16a) and FcγRIIIB (CD16b).

As used herein, the term 'oligomannose-type glycoform antibodies' refers to antibodies containing N-linked glycans in their Fc regions which are composed of only GlcNAc residues and mannose residues.

As used herein, the terms '*N*-glycan' and 'glycan' are used interchangeably and refer to an *N*-linked oligosaccharide, e.g. one that is or was attached by an *N-*acetylglucosamine residue linked to the amide nitrogen of an asparagine residue in a protein. The term 'glycoform' refers to a glycoprotein containing one or more types of glycan structures on the Fc domain. The predominant sugars found on glycoproteins are glucose, galactose, mannose, fucose, *N*-acetylgalactosamine (GalNAc), *N-*acetylglucosamine (GlcNAc) and sialic acid (e.g., *N*-acetyl-neuraminic acid (NANA)). The processing of the sugar groups occurs cotranslationally in the lumen of the ER and continues in the Golgi apparatus for *N*-linked glycoproteins.

*N*-glycans have a common pentasaccharide core of Man₃GlcNAc₂ ('Man' refers to mannose; 'Glc' refers to glucose and 'NAc' refers to *N*-acetyl; GlcNAc refers to *N-*acetylglucosamine). *N*-glycans differ with respect to the number of branches (antennae) comprising peripheral sugars (e.g. GlcNAc, galactose, fucose and sialic acid) that are added to the Man₃GlcNAc₂ ('Man3') core structure which is also referred to as the 'trimannose core', the 'pentasaccharide core' or the 'paucimannose core'. *N*-glycans are classified according to their branched constituents (e.g. high mannose-type, complex-type or hybrid-type). The term 'high-mannose' and 'oligomannose-type' are used interchangeably. A 'high mannose' type *N*-glycan has five or more mannose residues. A 'complex' type of *N*-glycan typically has at least one GlcNAc attached to the 1, 3 mannose arm and at least one GlcNAc attached to the 1, 6 mannose arm of a 'trimannose' core. Complex -type *N*-glycans may also have galactose ('Gal') or *N-*acetylgalactosamine ('GalNAc) residues that are optionally modified with sialic acid or derivatives (e.g. 'NANA' or 'NeuNAc' where 'Neu' refers to neuraminic acid and 'NAc' refers to *N*-acetyl). Complex *N*-glycans may also have intrachain substitutions comprising but not limited to 'bisecting' GlcNAc and core fucose ('fuc'). Complex *N-*glycans may also have multiple antennae on the 'trimannose core', often referred to as 'multiple antennary glycans.' A 'hybrid' type *N*-glycan has at least one GlcNAc on the terminal of the 1,3 mannose arm of the trimannose core and two or more mannoses on the 1,6 mannose arm of the trimannose core. Hybrid-type *N*-glycans may also have intrachain substitutions comprising but not limited to 'bisecting' GlcNAc and core fucose ('fuc'). Complex *N*-glycans may also have multiple antennae on the 'trimannose core', often referred to as 'multiple antennary glycans'. Glycoprotein molecules with common polypeptide chain but bearing different glycans are called "glycoforms" (Introduction to Glycobiology by ME Taylor and K Drickamer (OUP, 2011). ISBN 978-0-19-956911-3.). The terms "oligomannose-type", "hybrid-type" and "complex-type" glycans are established and refer both refer to the main categories of glycan structures and can also be used to describe glycoforms containing those categories of glycans (Molecular and Cellular Glycobiology Edited by Minoru Fukada and Ole Hindsgaul (OUP, 2000) ISBN 0 19 963807 1. Functional and Molecular Glycobiology by SA Brooks, MV Dwek and U Schumacher (BIOS Scientific Publishers Ltd, 2002) ISBN 1 85996 022 7. Introduction to Glycobiology by ME Taylor and K Drickamer (OUP, 2011). ISBN 978-0-19-956911-3.)

As used herein, the term 'bisecting *N*-acetylglucosamine' (bisecting GlcNAc) refers to a β-GlcNAc residue attached to the β-mannose of the trimannosyl core. The bisecting GlcNAc can be enzymatically attached to the trimannosyl core by a (1,4)- *N-*acetyl-glucosaminyltransferase III enzyme (GnTIII). CHO cells do not normally express GnTIII (Stanley et al. J. Biol. Chem. 261: 13370-13378 (1984)), but may be engineered to do so (Umana et al. Nature Biotech 17:176-180 (1999)).

Abbreviations used herein are of common usage in the art, see, e.g. abbreviations of sugars, above. Other common abbreviations include 'PNGase' or 'glycanase' which all refer to peptide *N*-glycosidase F (EC 3.2.2.18). Other common abbreviations include 'glycocosidase' which may refer to either an endoglycosidase or an exoglycosidase.

EndoS is an endoglycosidase secreted by the human pathogen *Streptococcus pyogenes.* EndoS specifically hydrolyses the asparagine-linked glycan on IgG between the two core GlcNAc residues. The EndoS polypeptide is preferably *S. pyogenes* EndoS, or a variant or fragment of *S. pyogenes* EndoS which retains IgG endoglycosidase activity. The variant may be an EndoS polypeptide from another organism, such as another bacterium. The bacterium is preferably a *Streptococcus,* such as *Streptococcus equi, Streptococcus zooepidemicus,* or preferably, *Streptococcus pyogenes.* Alternatively, the variant may be from *Cornyebacterium pseudotuberculosis,* for example the CP40 protein; *Enterococcus faecalis,* for example the EndoE protein; or *Elizabethkingia meningoseptica* (formerly *Flavobacterium meningosepticum*), for example the EndoF₂ from *Elizabethkingia meningoseptic* and CP40 from *Corynebacterium pseudotuberculosis.*

In one embodiment, the EndoS polypeptide may comprise:
(a) the amino acid sequence of SEQ ID NO: 1;
(b) a variant thereof having at least 50% identity to the amino acid sequence of SEQ ID NO: 1 and having IgG endoglycosidase activity; or
(c) a fragment of either (a) or (b) having IgG endoglycosidase activity.

Preferably, the EndoS polypeptide comprises, or consists of, the sequence of SEQ ID NO: 1. SEQ ID NO: 1 is the sequence of the mature form of EndoS, without the signal sequence and corresponds to amino acids 37 to 995 of SEQ ID NO: 2.

The polypeptide may additionally include a signal sequence. Accordingly, the EndoS polypeptide may comprise:
(a) the amino acid sequence of SEQ ID NO: 2;
(b) a variant thereof having at least 50% identity to the amino acid sequence of SEQ ID NO: 2 and having IgG endoglycosidase activity; or
(c) a fragment of either (a) or (b) having IgG endoglycosidase activity.
The EndoS polypeptide may consist of the sequence shown in SEQ ID NO: 2.

Variant polypeptides are those for which the amino acid sequence varies from that in SEQ ID NO: 1 or SEQ ID NO: 2, but which retain the same essential character or basic functionality as EndoS. The variant polypeptides may therefore display IgG endoglycosidase activity. Typically, polypeptides with more than about 50%, 55%, 60% or 65% identity, preferably at least 70%, at least 75%, at least 80%, at least 85%, at least 90% and particularly preferably at least 95%, at least 97% or at least 99% identity, with the amino acid sequence of SEQ ID NO: 1 or SEQ ID NO: 2 are considered variants of the protein. Such variants may include allelic variants and the deletion, modification or addition of single amino acids or groups of amino acids within the protein sequence, as long as the peptide maintains the basic functionality of EndoS. The identity of variants of SEQ ID NO: 1 or SEQ ID NO: 2 may be measured over a region of at least 100, at least 250, at least 500, at least 750, at least 800, at least 850, at least 900, at least 950, at least 955 or more contiguous amino acids of the sequence shown in SEQ ID NO: 1 or SEQ ID NO: 2, or more preferably over the full length of SEQ ID NO: 1 or SEQ ID NO: 2.

Variants of the amino acid sequence of SEQ ID NO: 1 preferably contain residues 191 to 199 of SEQ ID NO: 1, i.e., Leu-191, Asp-192, Gly-193, Leu-194, Asp-195, Val-196, Asp-107, Val-198, Glu-199, of SEQ ID NO:1 (which corresponds to residues 227 to 235 of SEQ ID NO:2, i.e. Leu-227, Asp-228, Gly-229, Leu-230, Asp-231, Val-232, Asp-233, Val-234 and Glu-235 of SEQ ID NO:2), These amino acids constitute a perfect chitinase family 18 active site, ending with glutamic acid. The glutamic acid in the active site of chitinases is essential for enzymatic activity. Most preferably, therefore, the variant of SEQ ID NO:1 contains Glu-199 of SEQ ID NO:1 and the variant of SEQ ID NO:2 contains Glu-235 of SEQ ID NO:2.

The fragment of the EndoS polypeptide used in the invention is typically at least 10, for example at least 20, 30, 40 or 50 more amino acids in length, up to 100, 200, 250, 300, 500, 750, 800, 850, 900, 950 or 955 amino acids on length, as long as it retains the IgG endoglycosidase activity of EndoS.

The endoglycosidase activity may be determined by means of a suitable assay. For example, a test polypeptide may be incubated with IgG at suitable temperature, such as 37°C. The starting materials and the reaction products may then be analysed by SDS PAGE. Typically, the molecular mass of the IgG heavy chain is reduced by approximately 3kDa if the test polypeptide has IgG endoglycosidase activity. Another assay for determining whether a test polypeptide has IgG endoglycosidase activity is by detection of glycosylated IgG using *Lens culinaris* agglutinin lectin (LCA), optionally using horseradish peroxidise and peroxidise substrate. Typically, the carbohydrate signal is reduced if the test polypeptide has IgG endoglycosidase activity. Another assay for determining whether a test polypeptide has IgG endoglycosidase activity is by incubation of a test polypeptide with purified IgG Fc fragments followed by reduction of the sample with 10mM dithiothreitol and mass spectroscopy (MALDI-TOF) analysis. Typically, the mass of monomeric IgG Fc is reduced by 1417 +14 Da if the test polypeptide has IgG endoglycosidase activity. Another assay for determining whether a test polypeptide has IgG endoglycosidase activity is to incubate the test polypeptide with IgG and test for released fragments of glycans. These fragments lack the reducing terminal GlcNAc residues and any potential fucose attached to that GlcNAc residues. These released carbohydrate structures can be detected by mass spectrometry (see for example Harvey DJ, et al. J Am Soc Mass Spectrom. 2011 Mar;22(3):568-81.) or can be detected by fluorescence labelling followed by HPLC equipped with a fluorescence detector (see for example Guile GR, et al.,Anal Biochem. 1996 Sep 5;240(2):210-26.).

Endo-beta-N-acetylglucosaminidase F3 (Endo F3) is an endoglycosidase originally isolated from *Flavobacterium meningosepticum* (Plummer TH Jr, Tarentino AL. Glycobiology. 1991 Jun;1(3):257-63.). Endo F3 cleaves free or Asparagine-linked triantennary or fucosylated biantennary oligosaccharides, as well as triamannosyl chitobiose core structures. It cleaves between the two N-acetylglucosamine residues in the diacetylchitobiose core of the oligosaccharide, generating a truncated sugar molecule with one N-acetylglucosamine residue remaining on the asparagine.

EndoF3 has no, or no significant activity to oligomannose-type glycans. Endoglycosidase Ebeta is the beta-domain of EndoE from *Enterococcus faecalis* that is similar to family 20 glycosyl hydrolases. (Collin M, Fischetti VA. J Biol Chem. 2004 May 21; 279(21):22558-70).)

Immunoglobulin G-degrading enzyme (IdeS) is an extracellular cysteine protease produced by the human pathogen *S. pyogenes* (von Pawel-Rammingen et al. EMBO J. 2002 April 2; 21(7): 1607-1615.). IdeS catalyses a single proteolytic cleavage in the lower hinge of human IgG (US7666582). IdeS also cleaves some subclasses of IgG in various animals and efficiently converts IgG into Fc and Fab fragments.

An assay for determining the sensitivity or resistance of IgG to IdeS or any other protease is to incubate IgG with the test protease and analyse the incubation mixture by SDS-PAGE. Cleavage of the IgG with the test protease will be evident by a reduction in the apparent molecular weight of the heavy chain. The cleaved IgG should exhibit at least a half of the binding to one or more Fc receptors.

Protein *N*-glycanase hydrolyses the amide linkage between GlcNAc and the Asn residue. An example of a protein *N*-glycanase is protein N-glycanase F (PNGase F).

As used herein, the terms "antibody", "immunoglobulin", "Ig" and "Ig molecule" are used interchangeably. Each antibody molecule has a unique structure that allows it to bind its specific antigen, but all antibodies/immunoglobulins have the same overall structure as described herein. The basic antibody structural unit is known to comprise a tetramer of subunits. Each tetramer has two identical pairs of polypeptide chains, each pair having one "light" chain (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Light chains are classified as either kappa or lambda. Heavy chains are classified as gamma, mu, alpha, delta, or epsilon, and define the antibody's isotype as IgG, IgM, IgA, IgD and IgE, respectively. The light and heavy chains are subdivided into variable regions and constant regions (See generally, Fundamental Immunology (Paul, W., ed., 2nd ed. Raven Press, N. Y., 1989), Ch. 7 (incorporated by reference in its entirety for all purposes). The variable regions of each light/heavy chain pair form the antibody binding site. Thus, an intact antibody has two binding sites. Except in bifunctional or bispecific antibodies, the two binding sites are the same. The chains all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. The terms include naturally occurring forms, as well as fragments and derivatives. Included within the scope of the term are classes of Igs, namely, IgG, IgA, IgE, IgM, and IgD. Also included within the scope of the terms are the subtypes of IgGs, namely, IgG1, IgG2, IgG3 and IgG4. The term is used in the broadest sense and includes single monoclonal antibodies (including agonist and antagonist antibodies) as well as antibody compositions which will bind to multiple epitopes or antigens. The terms specifically cover monoclonal antibodies (including full length monoclonal antibodies), polyclonal antibodies, multispecific antibodies (e.g., bispecific antibodies), and antibody fragments so long as they contain or are modified to contain at least the portion of the C_{H}2 domain of the heavy chain immunoglobulin constant region which comprises an *N*-linked glycosylation site of the C_{H}2 domain, or a variant thereof. Included within the terms are molecules comprising the Fc region, such as immunoadhesins (US Pat. Appl. No. 2004/0136986), Fc fusions and antibody-like molecules. Alternatively, these terms can refer to an antibody fragment of at least the Fab region that at least contains an *N*-linked glycosylation site.

The antibody may, for example, be a monoclonal antibody or a polyclonal antibody.

The term "Fc" fragment refers to the 'fragment crystallized' C-terminal region of the antibody containing the C_{H}2 and C_{H}3 domains. The term "Fab" fragment refers to the 'fragment antigen binding' region of the antibody containing the VH, CHl, VL and CL domains. As used herein, the term "Fc" fragment also includes the lower-hinge region between the cysteines of the interchain disulphide bonds and the Cγ2 domain.

The term "monoclonal antibody" (mAb) as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site.

Furthermore, in contrast to conventional (polyclonal) antibody preparations which typically include different antibodies directed against different determinants (epitopes), each mAb is directed against a single determinant on the antigen. In addition to their specificity, monoclonal antibodies are advantageous in that they can be synthesized by hybridoma culture, uncontaminated by other immunoglobulins. The term "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al, (1975) Nature, 256:495, or may be made by recombinant DNA methods (see, e.g., U.S. Pat. No. 4,816,567 to Cabilly et al.).

The antibodies herein include hybrid and recombinant antibodies produced by splicing a variable (including hypervariable) domain of an antibody with a constant domain (e.g. "humanized" antibodies), or a light chain with a heavy chain, or a chain from one species with a chain from another species, or fusions with heterologous proteins, regardless of species of origin or immunoglobulin class or subclass designation, (See, e.g., U.S. Pat. No. 4,816,567 to Cabilly et al.; Mage and Lamoyi, in Monoclonal Antibody Production Techniques and Applications, pp. 79-97 (Marcel Dekker, Inc., New York, 1987).) The antibodies herein specifically include "chimeric" antibodies (immunoglobulins) in which a portion of the heavy and/or light chain is identical with or homologous to corresponding sequences in antibodies derived from a first species or belonging to a particular antibody class or subclass, while the remainder of the chain(s) is identical with or homologous to corresponding sequences in antibodies derived from a different species or belonging to a different antibody class or subclass, as well as fragments of such antibodies, so long as they contain or are modified to contain at least one C_{H}2.

"Humanized" forms of non-human (e.g., murine) antibodies are specific chimeric immunoglobulins, immunoglobulin chains or fragments thereof (such as Fv, Fab, Fab', F(ab')₂, or other antigen-binding subsequences of antibodies) which contain sequences derived from human immunoglobulins. An Fv fragment of an antibody is the smallest unit of the antibody that retains the binding characteristics and specificity of the whole molecule. The Fv fragment is a noncovalently associated heterodimer of the variable domains of the antibody heavy chain and light chain. The F(ab)'2 fragment is a fragment containing both arms of Fab fragments linked by the disulfide bridges.

The most common forms of humanized antibodies are human immunoglobulins (recipient antibody) in which residues from a complementary-determining region (CDR) of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues.

Furthermore, humanized antibodies can comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the CDR regions are those of a human immunoglobulin consensus sequence. The humanized antibody optimally also will comprise at least a portion of an immunoglobulin constant region (Fc), typically that of a human immunoglobulin. For further details see Jones et al, 1986, Nature 321 :522-524; Reichmann et al, 1988, Nature 332:323-327, and Presta, 1992, Curr. Op. Struct. Biol. 2:593-596.

"Fragments" within the scope of the terms antibody or immunoglobulin include those produced by digestion with various proteases, those produced by chemical cleavage and/or chemical dissociation and those produced recombinantly, so long as the fragment remains capable of specific binding to a target molecule. Among such fragments are Fc, Fab, Fab', Fv, F(ab')₂, and single chain Fv (scFv) fragments.

Targets of interest for therapeutic antibodies for use in the method of the invention include CD2, CD3, CD19, CD20, CD22, CD25, CD30, CD33, CD40, CD52, CD56, CD64, CD70, CD74, CD79, CD80, CD86, CD105, CD138, CD174, CD205, CD227, CD326, CD340, MUC16, GPNMB, PSMA, Cripto, ED-B, TMEFF2, EphA2, EphB2, FAP, av integrin, Mesothelin, EGFR, TAG-72, GD2, CA1X, 5T4, α4β7 integrin, Her2. Other targets are cytokines, such as interleukins IL-I through IL- 13, tumour necrosis factors α & β, interferons α, β and γ, tumour growth factor Beta (TGF-β), colony stimulating factor (CSF) and granulocyte monocyte colony stimulating factor (GMCSF). See Human Cytokines: Handbook for Basic & Clinical Research (Aggrawal *et al.* eds., Blackwell Scientific, Boston, MA 1991). Other targets are hormones, enzymes, and intracellular and intercellular messengers, such as, adenyl cyclase, guanyl cyclase, and phospholipase C. Other targets of interest are leukocyte antigens, such as CD20, and CD33. Drugs may also be targets of interest. Target molecules can be human, mammalian or bacterial. Other targets are antigens, such as proteins, glycoproteins and carbohydrates from microbial pathogens, both viral and bacterial, and tumors. Still other targets are described in U.S. 4,366,241.

Examples of suitable antibodies include Abagovomab, Abciximab,Actoxumab, Adalimumab, Adecatumumab, Afelimomab, Afutuzumab, Alacizumab pegol, ALD518, Alemtuzumab, Alirocumab, Altumomab pentetate, Amatuximab, Anatumomab mafenatox, Anrukinzumab, Apolizumab, Arcitumomab, Aselizumab, Atinumab, Atlizumab (= tocilizumab), Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumab mertansine, Blinatumomab, Blosozumab, Brentuximab vedotin, Briakinumab, Brodalumab, Canakinumab, Cantuzumab mertansine, Cantuzumab ravtansine, Caplacizumab, Capromab pendetide, Carlumab, Catumaxomab, CC49, Cedelizumab, Certolizumab pegol, Cetuximab, Ch.14.18, Citatuzumab bogatox, Cixutumumab, Clazakizumab, Clenoliximab, Clivatuzumab tetraxetan, Conatumumab, Concizumab, Crenezumab, CR6261, Dacetuzumab, Daclizumab, Dalotuzumab, Daratumumab, Demcizumab, Denosumab, Detumomab, Dorlimomab aritox, Drozitumab, Duligotumab, Dupilumab, Dusigitumab, Ecromeximab, Eculizumab, Edobacomab, Edrecolomab, Efalizumab, Efungumab, Elotuzumab Elsilimomab, Enavatuzumab, Enlimomab pegol, Enokizumab, Enoticumab, Ensituximab, Epitumomab cituxetan, Epratuzumab, Erlizumab, Ertumaxomab, Etaracizumab, Etrolizumab, Evolocumab, Exbivirumab, Fanolesomab, Faralimomab Farletuzumab, Fasinumab, FBTA05, Felvizumab, Fezakinumab, Ficlatuzumab, Figitumumab, Flanvotumab, Fontolizumab, Foralumab, Foravirumab, Fresolimumab, Fulranumab, Futuximab, Galiximab,Ganitumab, Gantenerumab, Gavilimomab, Gemtuzumab ozogamicin, Gevokizumab, Girentuximab,Glembatumumab vedotin, Golimumab, Gomiliximab,GS6624, Ibalizumab, Ibritumomab tiuxetan, Icrucumab, Igovomab, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lampalizumab, Lebrikizumab, Lemalesomab, Lerdelimumab, Lexatumumab, Libivirumab, Ligelizumab, Lintuzumab, Lirilumab, Lodelcizumab, Lorvotuzumab mertansine, Lucatumumab, Lumiliximab, Mapatumumab, Maslimomab, Mavrilimumab, Matuzumab, Mepolizumab, Metelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Narnatumab, Natalizumab, Nebacumab, Necitumumab, Nerelimomab, Nesvacumab, Nimotuzumab, Nivolumab, Nofetumomab merpentan, Ocaratuzumab, Ocrelizumab, Odulimomab, Ofatumumab, Olaratumab, Olokizumab, Omalizumab, Onartuzumab, Oportuzumab monatox, Oregovomab, Orticumab, Otelixizumab, Oxelumab, Ozanezumab, Ozoralizumab, Pagibaximab, Palivizumab, Panitumumab, Panobacumab, Parsatuzumab, Pascolizumab, Pateclizumab, Patritumab, Pemtumomab, Perakizumab, Pertuzumab, Pexelizumab, Pidilizumab, Pinatuzumab vedotin, Pintumomab, Placulumab, Polatuzumab vedotin, Ponezumab, Priliximab, Pritoxaximab, Pritumumab, PRO 140, Quilizumab, Racotumomab, Radretumab, Rafivirumab, Ramucirumab, Ranibizumab,Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Seribantumab, Setoxaximab, Sevirumab, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tefibazumab, Telimomab aritox, Tenatumomab, Teneliximab, Teplizumab, Teprotumumab, TGN1412, Ticilimumab (= tremelimumab), Tildrakizumab, Tigatuzumab, TNX-650, Tocilizumab (= atlizumab), Toralizumab, Tositumomab, Tralokinumab, Trastuzumab, TRBS07, Tregalizumab, Tremelimumab Tucotuzumab celmoleukin, Tuvirumab, Ublituximab, Urelumab, Urtoxazumab, Ustekinumab, Vapaliximab, Vatelizumab, Vedolizumab, Veltuzumab,Vepalimomab Vesencumab, Visilizumab, Volociximab, Vorsetuzumab mafodotin, Votumumab, Zalutumumab, Zanolimumab, Zatuximab, Ziralimumab and Zolimomab aritox .

Preferred therapeutic antibodies that can be modified to become resistant to an agent which reduces Fc receptor binding of endogenous serum antibodies include Natalizumab, Vedolizumab, Belimumab, Atacicept, Alefacept, Otelixizumab, Teplizumab, Rituximab, Ofatumumab, Ocrelizumab, Epratuzumab, Alemtuzumab, Abatacept, Eculizamab, Omalizumab, Canakinumab, Meplizumab, Reslizumab, Tocilizumab, Ustekinumab, Briakinumab, Etanercept, Inlfliximab, Adalimumab, Certolizumab pegol, Golimumab, Trastuzumab, Gemtuzumab, Ozogamicin, Ibritumomab, Tiuxetan, Tostitumomab, Cetuximab, Bevacizumab, Panitumumab, Denosumab, Ipilimumab, Brentuximab and Vedotin.

Immune Fc receptors discussed herein, may include: FcyRI, FcyRIIa, FcyRIIb, FcyRIIIa, FcyRIIIb and FcRn (neonatal receptor). The term FcyRI can refer to any FcyRI subtype unless specified otherwise. The term FcyRII can refer to any FcyRII receptor unless specified otherwise. The term FcyRm refers to any FcyRIH subtype unless specified otherwise.

"Derivatives" within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Intracellular Antibodies: Research and Disease Applications, (Marasco, ed., Springer- Verlag New York, Inc., 1998).

The term 'peptide' as used herein refers to a short polypeptide e.g. one that is typically less than about 50 amino acids long and more typically less than about 30 amino acids long. The term as used herein encompasses analogs and mimetics that mimic structural and this biological function.

The term 'polypeptide' encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

The term 'isolated protein' or 'isolated polypeptide' is a protein or polypeptide that by virtue of its origin or source of derivation (1) is not associated with naturally associated components that accompany it in its native state, (2) exists in a purity not found in nature, where purity can be adjudged with respect to the presence of other cellular material (e.g. is free of other proteins from the same species) (3) is expressed by a cell from a different species, or (4) does not occur in nature (e.g. it is a fragment of a polypeptide found in nature or it includes amino acid analogs or derivatives not found in nature or linkages other than standard peptide bonds). Thus, a polypeptide that is chemically synthesised or synthesised in a cellular system different from the cell from which it naturally originates will be 'isolated' from its naturally associated components, A polypeptide or protein may also be rendered substantially free of naturally associated components by isolation, suing protein purification techniques well known in the art. As thus defined, 'isolated' does not necessarily require that the protein, polypeptide, peptide, or oligo peptide so described has been physically removed from it native environment.

The term 'polypeptide fragment' as used herein refers to a polypeptide that has a deletion, e.g. an amino-terminal and/or carboxy-terminal deletion compared to a full-length polypeptide. In a preferred embodiment, the polypeptide fragment is a contiguous sequence in which the amino acid sequence of the fragment is identical to the corresponding positions in the naturally-occurring sequence. Fragments typically are at least 5, 6, 7, 8, 9, or 10 amino acids long, preferably at least 12, 14, 16 or 18 amino acids long, more preferably at least 20 amino acids long, more preferably at least 25, 30, 35, 40, or 45 amino acids, even more preferably at least 50 or 60 amino acids long, and even more preferably at least 70 amino acids long.

A protein has 'homology' or is 'homologous' to a second protein if the nucleic acid sequence that encodes the protein has a similar sequence to the nucleic acid sequence that encodes the second protein. Alternatively, a protein has homology to a second protein if the two proteins have 'similar' amino acid sequences. (thus, the term 'homologous proteins' is defined to mean that the two proteins have similar amino acid sequences), In a preferred embodiment, a homologous protein is one that exhibits at least 65% sequence homology to the wild type protein, more preferred is at least 70% sequence homology. Even more preferred are homologous proteins that exhibit at least 75%, 80%, 85%, or 90% sequence homology to the wild type protein. In a yet more preferred embodiment a homologous protein exhibits at least 95%, 98%, 99% or 99.9% sequence identity. As used herein, homology between two regions of amino acid sequence (especially with respect to predicted structural similarities) is interpreted as implying similarity in function.

Oligomannose-type glycoform antibodies can be made by well established expression systems. Some illustrations of the approaches include the production of a heterogeneous mixture of oligomannose-type glycans utilising yeast expression systems such as *Pichia pastoris* put on a range of oligomannose-type glycans but typically Man₈₋₁₂GlcNAc₂ but can be larger or smaller. For example, a *P. Pastoris* cell line to express antibodies containing Man₅GlcNAc₂ has been reported by Potgieter TI, et al. J Biotechnol. 2009 Feb 23; 139(4):318-25. Epub 2008 Dec 27." A further example of antibody expression in yeast is described by Horwitz AH, et al. Chang CP, Better M, Hellstrom KE, Robinson RR in Proc Natl Acad Sci U S A. 1988 Nov;85(22):8678-82." Antibodies produced in this way exhibit enhanced ADCC consistent with the lack of fucose (Horwitz AH, et al. Proc Natl Acad Sci U S A. 1988 Nov; 85(22):8678-82). Another method involves expression in a mammalian cell line in the presence of an alpha 1,2-mannosidase inhibitor such as kifunensine (Elbein, Tropea et al. 1990; Chang, Crispin et al. 2007; Kanda, Yamada et al. 2007; Zhou, Shankara et al. 2008; van Berkel, Gerritsen et al. 2010) where the concentration is sufficient to give partial inhibition e.g. 1-5 microM in the HEK 293T system (Chang, Crispin et al. 2007).

Homogenous glycoforms of oligomannose-type antibodies such as the Man₉GlcNAc₂ form can be made via expression in a mammalian cell line in the presence of an alpha 1,2-mannosidase inhibitor such as kifunensine.(Elbein AD, et al. J Biol Chem. 1990 Sep 15;265(26):15599-605; Chang VT, et al., Structure. 2007 Mar;15(3):267-73) where the concentration is sufficient to give more homogenous glycoform e.g. >5 and ideally >10 microM in HEK 293T expression system. Kifunensine can also be used to generate oligomannose-type glycoforms of antibodies secreted by hybridoma cell lines or stably transfected or transiently transfected eukaryotic cell lines. An alternative method involves expression in a yeast cell line where the genetics of pathway have been altered to not process the glycan beyond Man₉GlcnAc₂ e.g. the type of pichia cell lines developed by GlycoFi (now Merck) (Li H, et al., Nat Biotechnol. 2006 Feb;24(2):210-5. Epub 2006 Jan 22).

The homogeneous Man₅GlcNAc₂ glycoform can be produced via expression in a mammalian cell line deficient in GlcNAc transferase I (GnT I) e.g CHO Lec1 or CHO Lec3.2.8. (Patnaik SK, Stanley P. Methods Enzymol. 2006;416:159-82. Review.), GnT I-deficient HEK 293S cells (Reeves, P. J., N. Callewaert, et al. (2002). Proc Natl Acad Sci U S A 99(21): 13419-13424) or alternatively by the engineered yeast expression systems as above (Potgieter TI, et al., J Biotechnol. 2009 Feb 23;139(4):318-25. Epub 2008 Dec 27).

The Man₈GlcNAc₂ glycoform can be produced using the Man₉GlcNAc₂ glycoform as above but with processing by ER mannosidase I e.g. post expression digestion (Dunlop DC, Bonomelli C, Mansab F, Vasiljevic S, Doores KJ, Wormald MR, Palma AS, Feizi T, Harvey DJ, Dwek RA, Crispin M, Scanlan CN. Glycobiology. 2010 Jul;20(7):812-23. Epub 2010 Feb 24.). In another method, the Man₈GlcNAc₂ glycoform can be produced utilising the engineered yeast expression systems as above. Further expression host systems found in the art for production of glycoproteins include: CHO cells (Raju WO9922764A1 and Presta WO03/035835A1); hybridoma cells (Trebak et al., 1999, J. Immunol. Methods, 230:59-70); insect cells (Hsu et al., 1997,JBC, 272:9062-970); and plant cells (Gerngross et al., WO04/07499A2). Endoglycosidase resistant glycoforms of IgG can be recombinantly produced by eukaryotic cell lines containing the glycosidase and glycosyltransferase activities. Eukaryotic cell lines can be engineered to have specific glycosidase and glycosyltransferase activities that generate a particular glycoform or glycoforms. For example, *Pichia pastoris* have been engineered to contain human glycosyltransferases. The resulting engineered *Pichia pastoris* cell lines secrete glycoproteins containing glycans not found in glycoproteins from native *Pichia pastoris* cell lines but they secret glycoproteins similar to mammalian glycoproteins. Similar mutant cell lines or organisms can be generated in other eukaryotic expression systems such as fly, plant, and mammalian cell lines. Using such engineered cell lines or organisms, endoglycosidase resistant glycoproteins can be generated. Using such engineered cell lines or organisms, glycoproteins containing triantennary or tetraantennary or pentaantennary glycans can be generated e.g. in yeast: Hamilton SR, Gerngross TU. Curr Opin Biotechnol. 2007 Oct;18(5):387-92. Epub 2007 Oct 24.. Additional methods for synthesising glycoforms include the use of glycosyltransferases and/or glycosidases to change the glycan composition of secreted glycoproteins.

Hybrid-type glycoforms can be produced using eukaryotic expressions systems deficient in Golgi α-mannosidase II activity. Cell lines can be genetically engineered to be deficient in Golgi α-mannosidase II activity. For example, the Lec36 cell line based on the human embryonic kidney 293T cell line have been generated by lectin-resistance (Crispin M et al 2009, Journal of Biological Chemistry, 284, 21684-21695). Glycoproteins expressed in the Lec36 cells contain hybrid-type glycosylation. Alternatively, engineered yeast cells can be constructed that contain mammalian glycosidase and glycosyltransferases but which lack Golgi α-mannosidase II activity. Additional methods for expressing glycoproteins containing hybrid-type glycans include the use of Golgi α-mannosidase II inhibitors such as swainsonine (Tulsiani DR, Harris TM, Touster O. J Biol Chem. 1982 Jul 25;257(14):7936-9; Crispin M et al 2009, Journal of Biological Chemistry, 284, 21684-21695).

Plant expression systems can also be engineered to produce defined glycoforms or range of glycoforms of glycoproteins. Strategies to modulate the glycosylation in plant expressions systems include targeting to the ER as well as glycosyltransferase and/or glycosidase gene knockouts and/or knock ins (Gomord V, Fitchette AC, Menu-Bouaouiche L, Saint-Jore-Dupas C, Plasson C, Michaud D, Faye L. Plant Biotechnol J. 2010 Jun;8(5):564-87).

Glycoforms containing "bisecting *N*-acetylglucosamine" wherein glycoform contains beta-*N*-acetylglucosamine 1-4 linked to a beta-mannose residue can be generated by expression in a eukaryotic expression system that is capable of producing complex-type or hybrid-type glycans and expresses GlcNAc transferase III (also known as GnTIII and UDP-N-acetylglucosamine: β-D mannoside β(1, 4)-N-acetylglucosaminyltransferase) (e.g. US 7897842).

Whilst EndoS can be used to deactivate competing serum antibodies, the engineered therapeutic antibody can also be deactivated using an endoglycosidase active to that antibody glycoform. For example, the use of oligomannose-type antibody glycoforms enables the therapeutic antibody to be deactivated with Endo H or Endo F1. This enables the deactivation of the therapeutic antibody to control the time of activity in the body or to deactivate it in the event of an adverse patient reactions.

As used herein, a glycoprotein composition "lacks" or "is lacking" a particular sugar residue, such as fucose or galactose, when no detectable amount of such sugar residue is present on the *N*-glycan structures at any time. For example, in preferred embodiments of the present invention, the glycoprotein compositions are produced by lower eukaryotic organisms, as defined above, including yeast (e.g., *Pichia sp*.; *Saccharomyces* sp.; *Kluyveromyces* sp.; *Aspergillus* sp.), and will "lack fucose," because the cells of these organisms do not have the enzymes needed to produce fucosylated N-glycan structures. Thus, the term "essentially free of fucose" encompasses the term "lacking fucose." However, a composition may be "essentially free of fucose" even if the composition at one time contained fucosylated N-glycan structures or contains limited, but detectable amounts of fucosylated N-glycan structures as described above.

The interaction of antibodies and antibody-antigen complexes with cells of the immune system and the variety of responses, including antibody-dependent cell-mediated cytotoxicity (ADCC) and complement-dependent cytotoxicity (CDC), clearance of immunocomplexes (phagocytosis), antibody production by B cells and IgG serum half-life are defined respectively in the following: Daeron et al, 1997, Annu. Rev. Immunol. 15: 203-234; Ward and Ghetie, 1995, Therapeutic Immunol. 2:77-94; Cox and Greenberg, 2001, Semin. Immunol. 13: 339-345; Heyman, 2003, Immunol. Lett. 88:157-161; and Ravetch, 1997, Curr. Opin. Immunol. 9: 121-125.

Antibodies can be incorporated into pharmaceutical compositions comprising the antibody as an active therapeutic agent and a variety of pharmaceutically acceptable components. See Remington's Pharmaceutical Science (15th ed., Mack Publishing Company, Easton, Pennsylvania, 1980). The preferred form depends on the intended mode of administration and therapeutic application. The compositions can include, depending on the formulation desired, pharmaceutically-acceptable, non-toxic carrier or diluents, which are defined as vehicles commonly used to formulate pharmaceutical compositions for animal or human administration. The diluent is selected so as not to affect the biological activity of the combination. Examples of such diluents are distilled water, physiological phosphate-buffered saline, Ringer's solutions, dextrose solution, and Hank's solution. In addition the pharmaceutical composition or formulation can also include other carriers, adjuvants, or nontoxic, nontherapeutic, nonimmunogenic stabilizers and the like.

The pharmaceutical carrier may be a liquid and the pharmaceutical composition would be in the form of a solution. Liquid carriers are used in preparing solutions, suspensions, emulsions, syrups, elixirs and pressurised compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilizers or osmo-regulators. Suitable examples of liquid carriers for oral and parenteral administration include water (partially containing additives e.g. cellulose derivatives, preferably sodium carboxymethyl cellulose solution), alcohols (including monohydric alcohols and polyhdric alcohols e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration.

Pharmaceutical compositions for parenteral administration are sterile, substantially isotonic, pyrogen-free and prepared in accordance with Good Manufacturing Practices (GMP) of the Food and Drug Administration (FDA) or similar body. Antibodies can be administered as injectable dosages of a solution or suspension of the substance in a physiologically acceptable diluent with a pharmaceutical carrier that can be a sterile liquid such as water, oils, saline, glycerol or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. Antibodies can be administered in the form of a depot injection or implant preparation which can be formulated in such a manner as to permit a sustained release of the active ingredient. Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polyactide, polyglycolide, or copolymer for enhanced adjuvant effect, as discussed above (see Langer, Science 249, 1527(1990) and Hanes, Advanced Drug Delivery Reviews 28, 97-119 (1997).

The agent and the therapeutic antibody may be administered by any suitable route. Preferably, they are both administered intravenously (i.v.).

Preferred embodiments of the present invention will now be described, merely by way of example, with reference to the following drawings and examples, wherein:
Figure 1 shows binding of human IgGI Fc to immobilized FcyRIIIa in the presence of PBS or increasing concentrations of human serum, treated or mock-treated with Endoglycosidase-S.
Figures 2A and 2B show mass spectrometric analysis of PNGase F release of *N-*linked glycans found on human serum IgG before EndoS digestion.
Figures 3A and 3B shows mass spectrometric analysis of PNGase F release of *N*-linked glycans found on human serum IgG after EndoS digestion. (Main ions are chloride adducts. Ions at *m*/*z* 1762 and 1924 are phosphate adducts; *m*/*z* 1519 is a fragment of *m*/*z* 1862.)
Figures 4A and 4B shows direct mass spectrometric analysis of glycans released by EndoS. (Ran as chloride adducts; m/z 1210, 1372 and 1534 are phosphate adducts; m/z 951, 1113 and 1275 are fragments.)

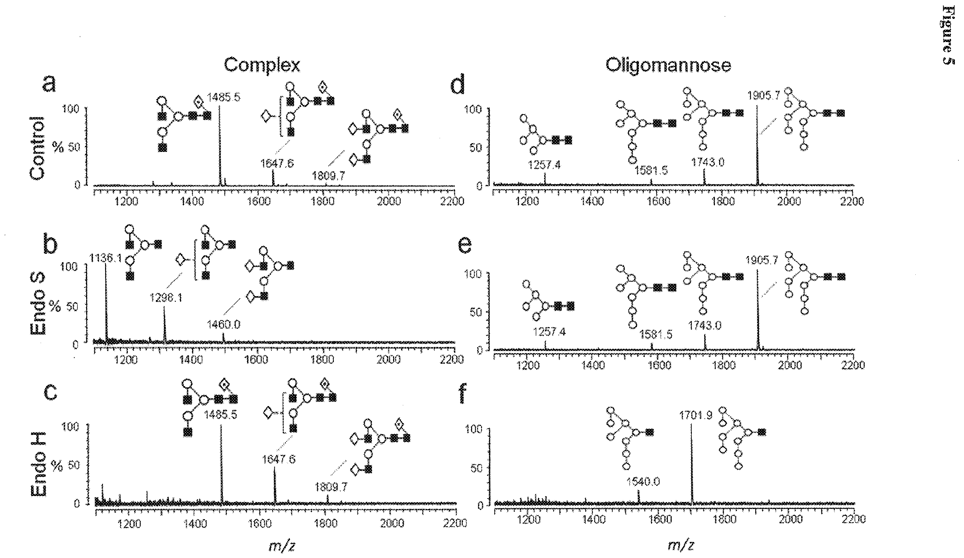

Figure 5 shows resistance of oligomannose-type containing Fc glycoforms to EndoS mediated hydrolysis. Naturally glycosylated IgG1 Fc (a-c) and oligomannose-type (Man₉GlcNAc₂) Fc (d-f) were digested with either Endo-S (b, e) or Endo-H (c, f). Glycan structures shown are based on the most abundant isomeric species, consistent with known biosynthetic pathways. The cleavage of the core GlcNAcβ1→4GlcNAc bond results in the removal of a single GlcNAc and attached Fucose residue from complex glycans (predicted Δ*m*/*z* = 349.1), and a GlcNAc from oligomannose-type-type glycans (predicted Δ*m*/*z* = 203.1).
Figure 6 shows the binding by ELISA of recombinant monoclonal IgG1 (mAb IgG CIIC1 which is a chimerical IgG with human Fc and mouse Fab) to immobilized FcγRIIIa is shown in the presence of buffer (PBS) or increasing concentrations of human serum. Binding was detected using a secondary antibody specific for the mouse Fab domain.
Figure 7 shows EndoS mediated deactivation of serum leads to enhancement of a mAb IgG CIIC1 (a chimerical IgG with human Fc and mouse Fab) binding to FcγRIIIa. The binding of human IgGI Fc containing oligomannose-type (Man₅GlcNAc₂) to FcγRIIIa is shown in the presence of PBS, serum, serum and Endo-S, serum and Endo-H, or serum and EndoS and Endo H. Data points represent the calculated mean of three independent measurements from a total of four experiments.
Figure 8 shows a schematic representation of how EndoS abolishes affinity of bulk serum antibodies to FcγRs but not glycan engineered (oligomannose-type) antibodies.

### Brief Description of the Sequences

SEQ ID NO: 1 is an amino acid sequence of EndoS isolated from *S. pyogenes* AP1.
SEQ ID NO: 2 is an amino acid sequence of EndoS isolated from *S. pyogenes* API, including a signal sequence.

### EXAMPLES

### The principle of the invention

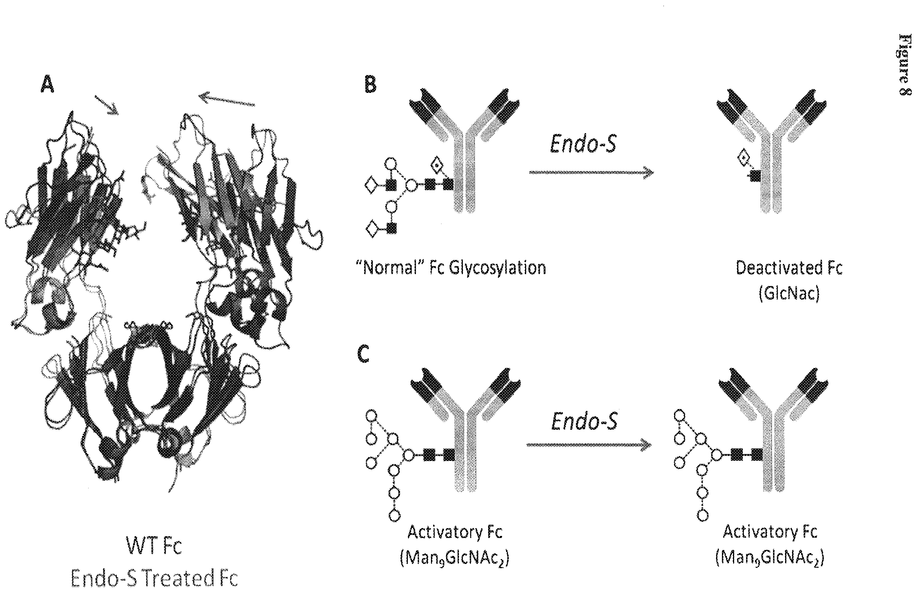

Figure 8 illustrates the principle of at least one aspect of the invention. In particular, Figure 8 shows a schematic representation of how EndoS abolishes affinity of bulk serum antibodies to FcγRs by removing the "normal" Fc glycosylation. However, EndoS has no effect on glycan engineered (oligomannose-type) antibodies, and activation of FcRs is achieved. Under physiological conditions the majority of FcRs are bound by "irrelevant", typically serum, Ig Fc. This places a hard limit on the effective concentration of available Fc receptors (FcγRs). In this embodiment Endo-S is used to significantly reduce the affinity of irrelevant serum antibodies to FcγRs but not of glycan engineered (oligomannose-type) monoclonal antibodies.

### Example 1: Reduction of serum IgG binding to FcγRIIIa increases the binding of resistant therapeutic antibody

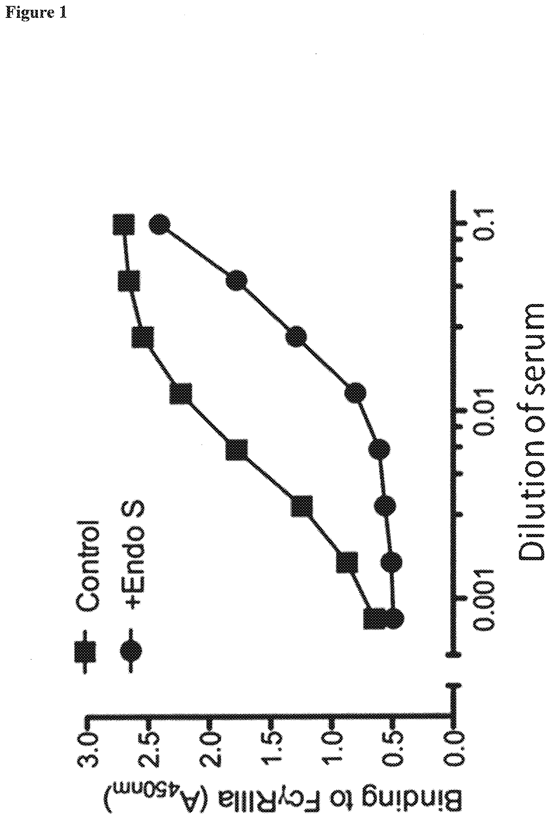

In order to demonstrate that EndoS could be used to reduce serum IgG binding to FcγRIIIa the following experiment was performed. FcγRIIIa (158Val variant; R&D systems, Minneapolis, U.S.A.) at 2.5 µg/mL in PBS was coated on high-binding microtitre plates (3690, Corning, NY, U.S.A.) overnight at 4°C. Coated plates were washed with PBS containing 0.05% Tween 20 (Sigma-aldrich, U.S.A.) and blocked for 2 hours at room temperature with 3% BSA in PBS. Serial dilutions of human serum (H4522, Sigma-Aldrich, U.S.A.) or recombinant human IgG1 glycoforms bearing Man₉GlcNAc₂ or Man₅GlcNAc₂ (starting concentration of 0.1 mg/mL in PBS), was then added and allowed to bind for 2 hours at room temperature. Plates were washed five times with PBS containing 0.05% Tween and binding was detected using a HRP conjugated Fab fragment specific for murine IgG Fab (ab98659, Abcam, Cambridge, UK). TMB substrate (Thermo Scientific, Rockford, IL, U.S.A.) was used for colour development according to manufacturer's directions. Colour development was stopped by the addition of 2M H₂SO₄ and absorbance was measured at 450 nm on a Spectramax M5 (Molecular Devices, California, U.S.A.) multiwall plate reader. Serum was incubated overnight with 1µg/mL of EndoS or PBS at 37°C. Control serum samples were mock treated with PBS and incubated overnight at 37°C. Data was processed and plotted using Prism (GraphPad software, California, U.S.A.). Apparent affinity was calculated as the concentration of oligomannose-type mAb corresponding to half-maximal binding on the ELISA binding curve. The binding of normal human sera was detected using labelled anti-human Fab. The results are shown in Figure 1 and clearly demonstrate that EndoS treatment of human serum IgG results in a decrease in FcγRIIIa binding.

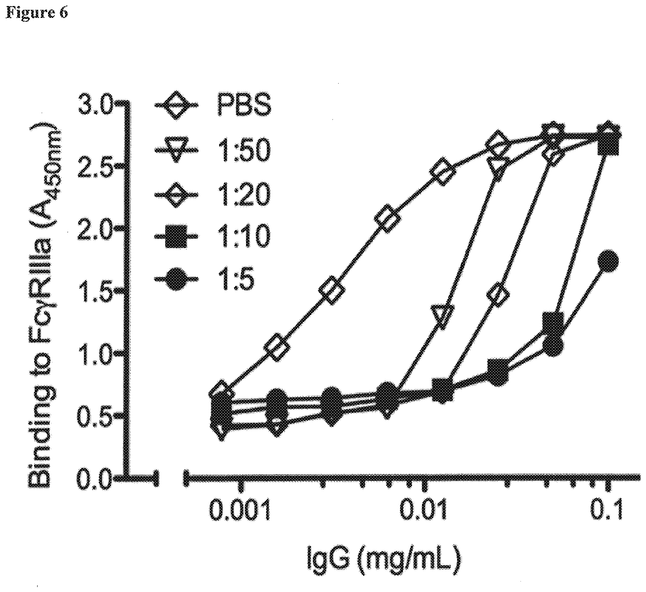

Figure 6 demonstrates that reduced binding of a monoclonal antibody IgG CIIC1 (a chimerical IgG with human Fc, and mouse Fab which enables specific detection of the monoclonal against the larger pool of serum antibodies) to FcγRIIIa is observed with increasing concentrations of serum IgG. The binding was determined at increasing dilutions of sera (1:5, 1:10, 1:20 and 1:50) and in PBS for a range of monoclonal IgG concentrations (x-axis). Binding of the monoclonal antibody IgG CIIC1 was detected using HRP-conjugated anti-mouse (Fab)'2 antibody. Figure 6 shows how serum IgG outcompetes thes monoclonal antibody IgG CIIC1.

### Serum IgG is sensitive to EndoS activity

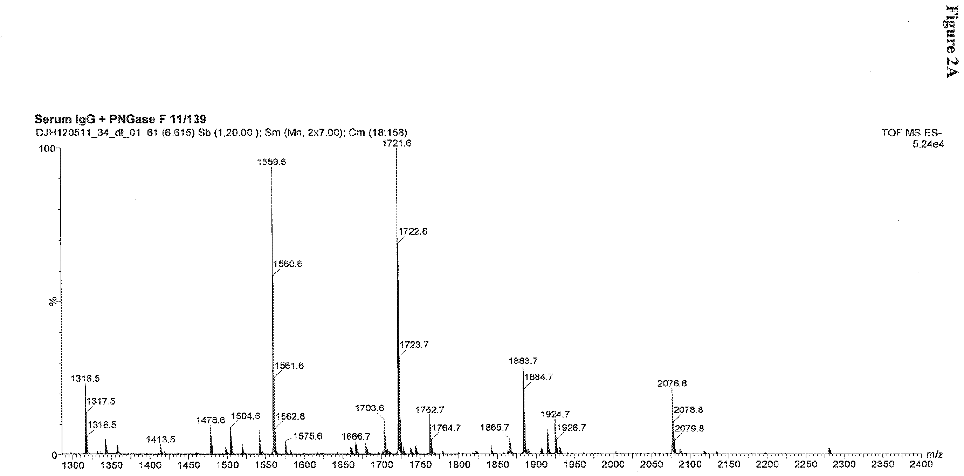

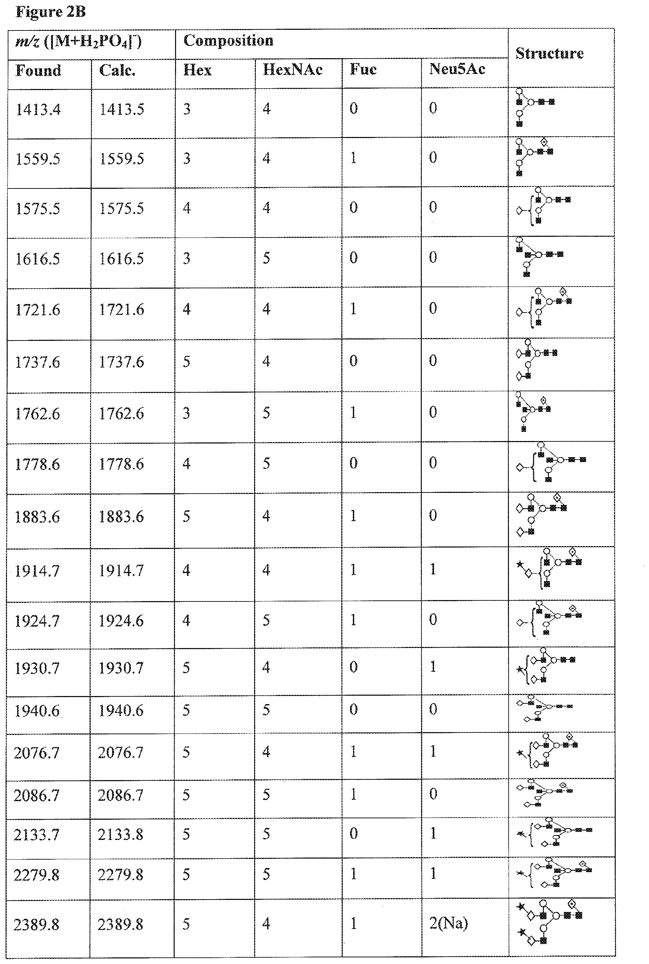

In order to demonstrate that bisected glycans and sialylated structures were resistant to EndoS, compositional analysis of *N*-linked glycans found on human serum IgG was performed before (a) and after (b) EndoS digestion as described in the Materials and Methods section. The major, neutral bi-antennary structures found on native serum Ig (m/z at 1559, 1721, 1883) were entirely absent on IgG that has been exposed to EndoS. In contrast the corresponding bisected structures (m/z 1762, 1925, and 2087 in Figures 2A and 2B) represented a minor population prior to digestion with Endo-S but corresponded to the most abundant species on IgG following EndoS digestion.

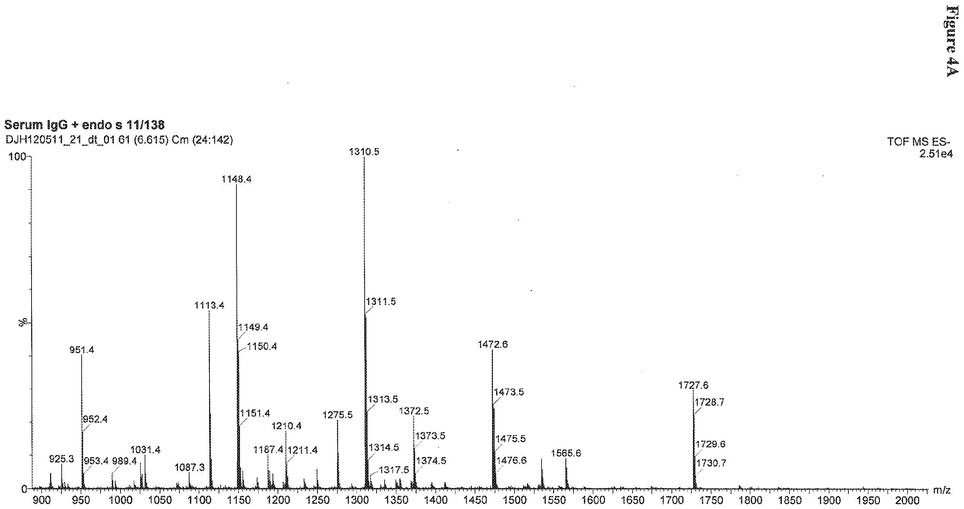

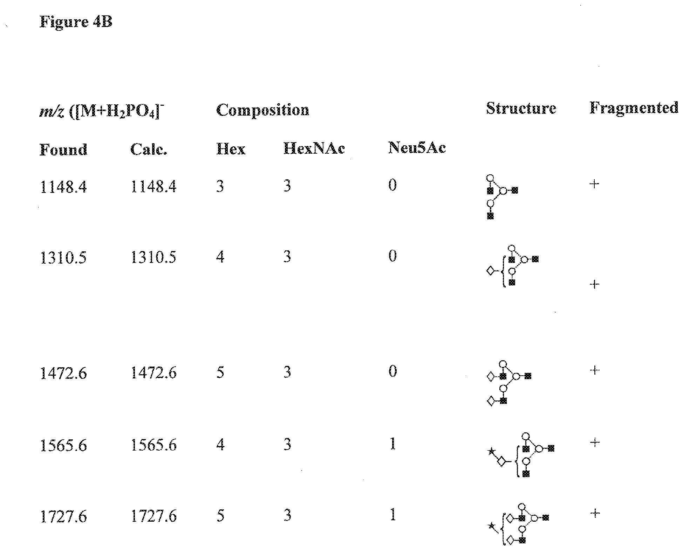

Direct mass spectrometric analysis of the glycans released by EndoS was performed as described in the Materials and Methods Section. Figures 4A and 4B shows that neutral bi-antennary structures were hydrolysed (yielding products at m/z 1148, 1310, 1473) but no detectable bisecting structures were found in the released pool. Some mono-sialylated structures were found in the released pool (m/z 1566 and 1728) but were found as an enriched fraction on digested IgG (m/z 2078 and 2280), indicating these structures showed partial resistance to EndoS. Consistent with the resistance of neutral, bisected structures, the bisected, sialylated structure (m/z 2280) was significantly enriched as a fraction of residual glycans. Most of the sialylated glycans were released with EndoS.

Overall these data show bisected glycans are resistant to EndoS, sialylated structures are moderately resistant and the major population of bi-antennary glycans on IgG (lacking bisects and sialic acids) are entirely sensitive to EndoS.

### Ability of oligomannose-type antibodies to resist enzymatic degradation by EndoS

To demonstrate that oligomannose-type antibodies could be generated that were resistant to EndoS the following experiments were performed.

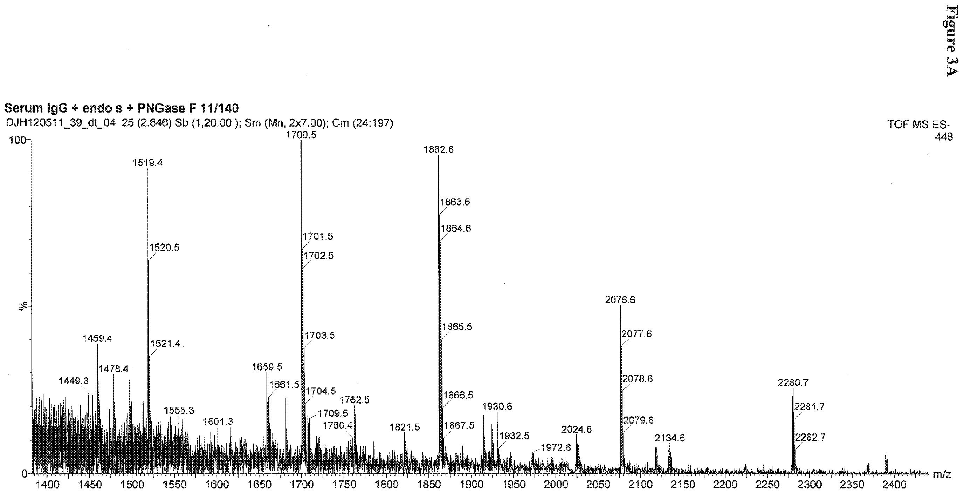

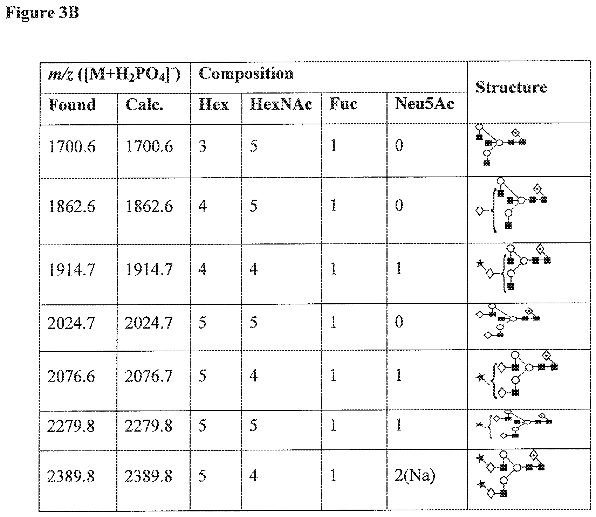

Figure 5 shows the resistance of oligomannose-type Fc glycoforms to EndoS mediated hydrolysis. Recombinant IgG-Fc domain was expressed in human embryonic kidney 293T cells either without Figure 3 (a) or with (d) the inhibitor kifunensine as described in the Materials and Methods section. This produces a typical biantennary, complex-type glycan profile as shown in (a) similar to the profile seen in human sera as illustrated in Figures 2A and 2B or produces a purely oligomannose-type profile (d) if the inhibitor kifunensine is used. Figure 5(b) shows how EndoS was able to completely cleave the complex-type glycans but had no effect on the oligomannose-type glycan (e). Digestion with EndoH demonstrated the reciprocal specificity of EndoH and how it is unable to cleave complex-type glycans as shown in Figure 5(c) with complete hydrolysis seen of the oligomannose-type Fc glycoform as shown in Figure 5(f).

In order to demonstrate that EndoS could be used to increase the apparent affinity of an oligomannose-type-mAb for FcγRIIIa the following experiment was performed. FcγRIIIa (158Val variant; R&D systems, Minneapolis, U.S.A.) at 2.5 µg/mL in PBS was coated on high-binding microtitre plates (3690, Corning, NY, U.S.A.) overnight at 4°C. Coated plates were washed with PBS containing 0.05% Tween 20 (Sigma-aldrich, U.S.A.) and blocked for 2 hours at room temperature with 3% BSA in PBS. Serial dilutions of human serum (H4522, Sigma-Aldrich, U.S.A.) or recombinant human IgG1 glycoforms bearing Man₉GlcNAc₂ or Man₅GlcNAc₂ (starting concentration of 0.1 mg/mL in PBS), was then added and allowed to bind for 2 hours at room temperature. Plates were washed five times with PBS containing 0.05% Tween and binding was detected using a HRP conjugated Fab fragment specific for murine IgG Fab (ab98659, Abcam, Cambridge, UK). TMB substrate (Thermo Scientific, Rockford, IL, U.S.A.) was used for colour development according to manufacturer's directions. Colour development was stopped by the addition of 2M H₂SO₄ and absorbance was measured at 450 nm on a Spectramax M5 (Molecular Devices, California, U.S.A.) multiwall plate reader. A 1:5 dilution of serum was incubated with 1:100 dilution of EndoS (1 mg/mL) or Endo H (500 U/µL) overnight at 37°C Control serum samples were mock treated with PBS and incubated overnight at 37°C. Data was processed and plotted using Prism (GraphPad software, California, U.S.A.). Apparent affinity was calculated as the concentration of oligomannose-type mAb corresponding to half-maximal binding on the ELISA binding curve.

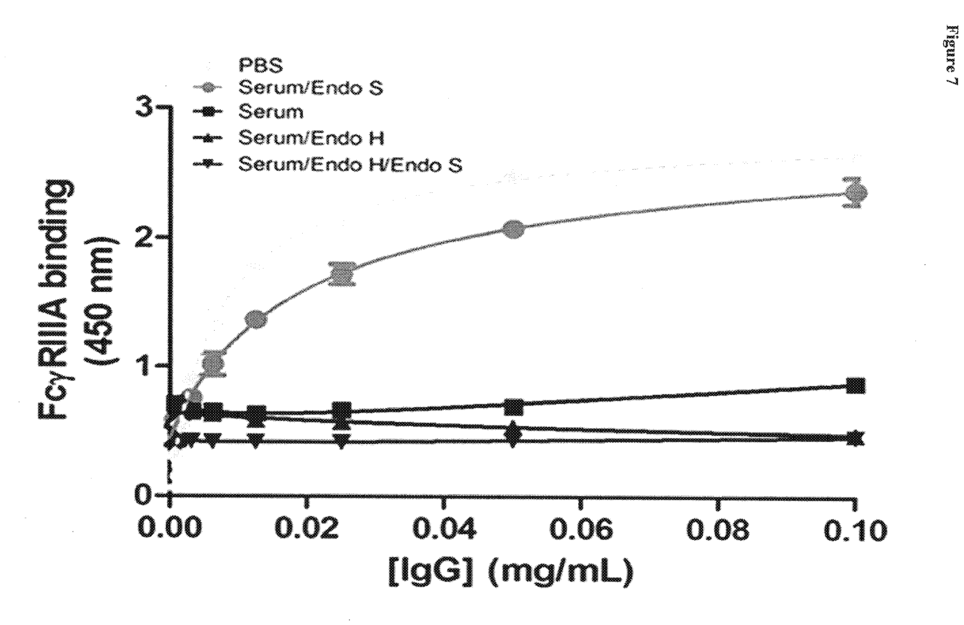

Consistent with the data from Figure 1, Figure 7 shows that the enzyme-free serum efficiently blocked the binding of the oligomannose-type mAb IgG CIIC1 to FcγRIIIa detected using HRP-conjugated anti-mouse Fab IgG. The binding was determined in the presence of only PBS, or in the presence of serum or serum plus combinations of EndoS and EndoH. In the absence of any endoglycosidase, the serum effectively outcompetes the oligomannose-type mAb IgG CIIC1. However, the addition of EndoS led to a dramatic increase in apparent affinity of oligomannose-type-mAb for FcγRIIIa with 50% receptor saturation achieved at approximately 0.05 µM of mAb, a level approaching that of mAb:FcR determined for IgG in the complete absence of serum and consistent with the reported value (0.08µM) of this interaction (Kanda, Y., Yamada, T., Mori, K., Okazaki, A., Inoue, M., Kitajima-Miyama, K., Kuni-Kamochi, R., Nakano, R., Yano, K., Kakita, S., Shitara, K. & Satoh, M. (2007). Glycobiology 17, 104-18).

This enhancement was a direct consequence of the differential glycosylation of the engineered monoclonal and natural serum Ab. This glycoform-dependence was confirmed by the addition of Endo H which led to a loss of detectable FcγRIIIa binding regardless of whether or not the competing sera had also been treated with EndoS. For example, the use of oligomannose-type antibody glycoforms enables the therapeutic antibody to be deactivated with Endo H as demonstrated in Figure 7. EndoH could be utilised for the deactivation of the therapeutic antibody to control the time of activity in the body or to deactivate it in the event of an adverse patient reactions.

### Materials and Methods

### Protein Expression and Purification of IgG1 oligomannose-type glycoforms Man₉GlcNAc₂ and Man₅GlcNAc₂.

Human IgG1 Fc (residues 240-440, following the numbering of Edelman *et al*.; GenBank accession no. J00228) was cloned into the pHLsec vector and transiently expressed in Human Embryonic Kidney cells as previously described (Aricescu et al. Acta Crystallogr D Biol Crystallogr. 2006 Oct;62(Pt 10):1243-50. Epub 2006 Sep 19) with DNA mixed with polyethyleneimine (PEI) in a mass ratio of 1:1.5, respectively. The Man₉GlcNAc₂ glycoform was obtained by transient expression in Human Embryonic Kidney 293T cells in the presence of 5 µM kifunensine, a class I α-mannosidase inhibitor (Chang VT, Crispin M, Aricescu AR, Harvey DJ, Nettleship JE, Fennelly JA, Yu C, Boles KS, Evans EJ, Stuart DI, Dwek RA, Jones EY, Owens RJ, Davis SJ. Structure. 2007 Mar;15(3):267-73), to generate IgG-Fc bearing the immature oligomannose-type N-linked glycan, Man₉GlcNAc₂. The Man₅GlcNAc₂ glycoform was obtained by transient expression in GlcNAc Transferase I-deficient Human Embryonic Kidney 293S cells Reeves, P. J., N. Callewaert, et al. (2002). Proc Natl Acad Sci U S A 99 (21): 13419-13424. Cell supernatant was clarified five days following transfection and IgG-Fc was purified by immobilized metal affinity chromatography using Chelating Sepharose Fast Flow Ni²⁺-agarose beads (GE Healthcare, Buckinghamshire, UK). IgG-Fc was partially deglycosylated at 25 °C for 12 h using 75 µg mL⁻¹ endoglycosidase H and then purified by size exclusion chromatography. Protein purity was assessed by SDS-PAGE analysis and typical yields of deglycosylated IgG were 20 mg L⁻¹ cell culture.

Full length IgG1 antibodies bearing human IgG1 Fc domains was generated by cloning the Fab domains of the murine monoclonal antibody CIIC1 (Developmental Studies Hybridoma Bank, University of Iowa, Department of Biology, Iowa City, IA 52242) into pFUSE-CHIg-hG1 vector (Invivogen, San Diego, California, U.S.A.). Intact CIIC1 antibody with complex and oligomannose-type glycans was transiently expressed in Human Embryonic Kidney cells as described above. Successful expression of full length antibody was confirmed by SDS-PAGE analysis and also by ELISA assay for binding to mouse collagen Type II protein.

### Enzymatic release of N-linked glycans.

Oligosaccharides were released from bands containing approximately 10 µg of target glycoprotein that were excised from Coomassie blue-stained reducing SDS-PAGE gels, washed with alternating water and acetonitrile and dried in a vacuum centrifuge, followed by rehydration with 100 Units/ml of PNGase F (New England Biolabs, MA, U.S.A.) and incubation for 12 hours at 37°C. The enzymatically released *N*-linked glycans were eluted with water. Endoglycosidase digestion of glycans was performed by addition of 1 µg of recombinant EndoS (Purchased from Genovis AB, Lund, Sweden and also obtained from Professor Ben Davis, CRL, University of Oxford) or 1µl of Endo H (500U/µl, New England Biolabs, MA, U.S.A.) and incubation for 12 hours at 37°C.

### Matrix-assisted laser desorption/ionization (MALDI) time-of-flight (TOF) mass spectrometry.

Aqueous solutions of the glycans generated by the aforementioned method were cleaned with a Nafion 117 membrane. Positive ion MALDI-TOF mass spectra were recorded with a Shimazu AXIMA TOF MALDI TOF/TOF fitted with delayed extraction and a nitrogen laser (337 nm). The acceleration voltage was 20 kV; the pulse voltage was 3200 V; and the delay for the delayed extraction ion source was 500 ns. Samples were prepared by adding 0.5 µL of an aqueous solution of the sample to the matrix solution (0.3 µL of a saturated solution of 2, 5-dihydroxybenzoic acid in acetonitrile) on the stainless steel target plate and allowing it to dry at room temperature. The sample/matrix mixture was then recrystallized from ethanol.

### Example 2: Mouse model

Cells from the cell line SKBR3 (a cell line with high expression of HER2) are introduced subcutaneously into mice. EndoS and a therapeutic antibody resistant to EndoS and directed towards HER2 (e.g. Herceptin) are introduced intravenously at a dose of 30mg/Kg week, for a period of 4 weeks. Control mice do not receive either (i) Endo S or (ii) the therapeutic antibody. The size of resultant tumours is measured by calipers.

### Example 3: Delayed administration of therapeutic antibody

A breast cancer subject is treated with 15mg EndoS *i.v.* in a saline solution. Patient IgG glycosylation levels are monitored by purification of a sample of patient IgG and assessment of the IgG mass by SDS-PAGE. (Deglycosylation of IgG results in a reduction of mass of the IgG).

After a delay of 2 days, the subject is treated with 2mg therapeutic antibody/kg body weight trastuzumab i.v.

### Example 4: Extracorporeal treatment of blood

The blood of a breast cancer subject is passed through an Endo S column at a rate of 250 mL/hour for 4 hours and returned to the subject. In this time, the glycosylated endogenous serum IgG levels drop to below 50% of the starting levels. The subject is subsequently treated with 2 mg/Kg trastuzumab i.v.

## Claims

1. An agent which reduces Fc receptor binding by enzymatic degradation of endogenous serum antibodies for use in a method of treating a disease, wherein the agent is an endoglycosidase, a protease or a protein-N-glycanase and the method increases the therapeutic potency of an antibody, wherein the mode of action of said antibody relies on the Fc:FcR interaction, and wherein the method comprises the steps:
(a) administering said agent to a subject; and subsequently,
(b) after a set time interval, administering said antibody to the subject.

2. The agent for use of claim 1, wherein the disease is cancer, infection or autoimmunity.

3. The agent for use of claim 2, wherein the cancer is Acute lymphoblastic leukemia, Acute myeloid leukemia, Adrenocortical carcinoma, AIDS-related cancers, AIDS-related lymphoma, Anal cancer, Appendix cancer, Astrocytoma, childhood cerebellar or cerebral, Basal cell carcinoma, Bile duct cancer, extrahepatic, Bladder cancer, Bone cancer, Osteosarcoma/Malignant fibrous histiocytoma, Brainstem glioma, Brain cancer, Brain tumor, cerebellar astrocytoma, Brain tumor, cerebral astrocytoma/malignant glioma, Brain tumor, ependymoma, Brain tumor, medulloblastoma, Brain tumor, supratentorial primitive neuroectodermal tumors, Brain tumor, visual pathway and hypothalamic glioma, Breast cancer, Bronchial adenomas/carcinoids, Burkitt lymphoma, Carcinoid tumor, Carcinoid tumor, gastrointestinal, Carcinoma of unknown primary, Central nervous system lymphoma, Cerebellar astrocytoma, Cerebral astrocytoma/Malignant glioma, Cervical cancer, Chronic lymphocytic leukemia, Chronic myelogenous leukemia Chronic myeloproliferative disorders, Colon Cancer, Cutaneous T-cell lymphoma, Desmoplastic small round cell tumor, Endometrial cancer, Ependymoma, Esophageal cancer, Ewing's sarcoma in the Ewing family of tumors, Extracranial germ cell tumor, Childhood, Extragonadal Germ cell tumor, Extrahepatic bile duct cancer, Eye Cancer, Intraocular melanoma, Eye Cancer, Retinoblastoma, Gallbladder cancer, Gastric (Stomach) cancer, Gastrointestinal Carcinoid Tumor, Gastrointestinal stromal tumor (GIST), Germ cell tumor: extracranial, extragonadal, or ovarian, Gestational trophoblastic tumor, Glioma of the brain stem, Glioma, Childhood Cerebral Astrocytoma, Glioma, Childhood Visual Pathway and Hypothalamic, Gastric carcinoid, Hairy cell leukemia, Head and neck cancer, Heart cancer, Hepatocellular (liver) cancer, Hodgkin lymphoma, Hypopharyngeal cancer, Hypothalamic and visual pathway glioma, Intraocular Melanoma, Islet Cell Carcinoma (Endocrine Pancreas), Kaposi sarcoma, Kidney cancer (renal cell cancer), Laryngeal Cancer, Leukemias, Leukemia, acute lymphoblastic (also called acute lymphocytic leukemia), Leukemia, acute myeloid (also called acute myelogenous leukemia), Leukemia, chronic lymphocytic (also called chronic lymphocytic leukemia), Leukemia, chronic myelogenous (also called chronic myeloid leukemia), Leukemia, hairy cell, Lip and Oral Cavity Cancer, Liposarcoma, Liver Cancer (Primary), Lung Cancer, Non-Small Cell ,Lung Cancer, Small Cell, Lymphomas, Lymphoma, AIDS-related, Lymphoma, Burkitt, Lymphoma, cutaneous T-Cell, Lymphoma, Hodgkin, Lymphomas, Non-Hodgkin (an old classification of all lymphomas except Hodgkin's), Lymphoma, Primary Central Nervous System, Macroglobulinemia, Waldenstrom, Malignant Fibrous Histiocytoma of Bone/Osteosarcoma, Medulloblastoma, Melanoma, Melanoma, Intraocular (Eye), Merkel Cell Carcinoma, Mesothelioma, Adult Malignant, Mesothelioma, Metastatic Squamous Neck Cancer with Occult Primary, Mouth Cancer, Multiple Endocrine Neoplasia Syndrome, Multiple Myeloma/Plasma Cell Neoplasm, Mycosis Fungoides, Myelodysplastic Syndromes, Myelodysplastic/Myeloproliferative Diseases, Myelogenous Leukemia, Chronic, Myeloid Leukemia, Adult Acute, Myeloid Leukemia, Childhood Acute, Myeloma, Multiple (Cancer of the Bone-Marrow), Myeloproliferative Disorders, Nasal cavity and paranasal sinus cancer, Nasopharyngeal carcinoma, Neuroblastoma, Non-Hodgkin lymphoma, Non-small cell lung cancer, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, Pancreatic cancer, islet cell, Paranasal sinus and nasal cavity cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Plasma cell neoplasia/Multiple myeloma, Pleuropulmonary blastoma, Primary central nervous system lymphoma, Prostate cancer, Rectal cancer, Renal cell carcinoma (kidney cancer), Renal pelvis and ureter, transitional cell cancer, Retinoblastoma, Rhabdomyosarcoma, Salivary gland cancer, Sarcoma, Ewing family of tumors, Kaposi Sarcoma, Sarcoma, soft tissue, Sarcoma, uterine, Sézary syndrome, Skin cancer (nonmelanoma), Skin cancer (melanoma), Skin carcinoma, Merkel cell, Small cell lung cancer, Small intestine cancer, Soft tissue sarcoma, Squamous cell carcinoma, Squamous neck cancer with occult primary, metastatic, Stomach cancer, Supratentorial primitive neuroectodermal tumor, T-Cell lymphoma, cutaneous - see Mycosis Fungoides and Sézary syndrome, Testicular cancer, Throat cancer, Thymoma, Thymoma and Thymic carcinoma, Thyroid cancer, Thyroid cancer, Transitional cell cancer of the renal pelvis and ureter, Trophoblastic tumor, Ureter and renal pelvis, transitional cell cancer Urethral cancer, Uterine cancer, endometrial, Uterine sarcoma, Vaginal cancer, Visual pathway and hypothalamic glioma, Vulvar cancer, Waldenstrom macroglobulinemia and Wilms tumor (kidney cancer).

4. The agent for use of claim 2, wherein the cancer is bladder cancer, lung cancer, breast cancer, melanoma, colon cancer, rectal cancer, non-Hodgkin's lymphoma, endometrial cancer, pancreatic cancer, kidney (renal cell) cancer, prostate cancer, leukemia, thyroid cancer and oesophageal cancer, preferably breast cancer.

5. The agent for use of any one of claims 1 to 4, wherein the agent and antibody are present as a combined preparation for sequential use.

6. The agent for use of any one of claims 1 to 5, wherein the set time interval is 1-2, 1-5, 1-10 or 1-20 days.

7. The agent for use of any one of claims 1 to 6, wherein the method comprises the steps:
(a) treating blood from the subject *ex vivo* with the agent;
(b) returning the treated blood to the subject; and subsequently
(c) administering said antibody to the subject.

8. The agent for use of any one of claims 1 to 7, wherein the endoglycosidase is endoglycosidase S (EndoS) or endoglycosidase F3 or endoglycosidase Ebeta, or wherein the protease is IdeS.

9. The agent for use of claim 8 wherein the agent is EndoS or IdeS.

10. The agent for use of any one of claims 1 to 9 wherein said antibody is Natalizumab, Vedolizumab, Belimumab, Atacicept, Alefacept, Otelixizumab, Teplizumab, Rituximab, Ofatumumab, Ocrelizumab, Epratuzumab, Alemtuzumab, Abatacept, Eculizamab, Omalizumab, Canakinumab, Meplizumab, Reslizumab, Tocilizumab, Ustekinumab, Briakinumab, Etanercept, Inlfliximab, Adalimumab, Certolizumab pegol, Golimumab, Trastuzumab, Gemtuzumab, Ozogamicin, Ibritumomab, Tiuxetan, Tostitumomab, Cetuximab, Bevacizumab, Panitumumab, Denosumab, Ipilimumab, Brentuximab and Vedotin.

11. The agent for use of claim 10, wherein said antibody is Trastuzumab.

## Patentansprüche

1. Mittel, das eine Fc-Rezeptorbindung durch enzymatischen Abbau von endogenen Serumantikörpern zur Verwendung in einem Verfahren zur Behandlung einer Krankheit reduziert, wobei das Mittel eine Endoglykosidase, eine Protease oder eine Protein-N-Glykanase ist und das Verfahren die therapeutische Wirksamkeit eines Antikörpers erhöht, wobei die Wirkungsweise des Antikörpers auf der Fc:FcR-Interaktion beruht, und wobei das Verfahren die folgenden Schritte umfasst:
a) Verabreichen des Mittels an ein Subjekt; und anschließend,
b) nach einem festgelegten Zeitintervall, Verabreichung des Antikörpers an das Subjekt.

2. Mittel zur Verwendung nach Anspruch 1, wobei die Krankheit Krebs, Infektion oder Autoimmunität ist.

3. Mittel zur Verwendung nach Anspruch 2, wobei der Krebs akute lymphatische Leukämie, akute myeloische Leukämie, Nebennierenkarzinom, AIDSbezogene Krebserkrankungen, AIDS-bezogenes Lymphom, Analkrebs, Appendix-Krebs, Astrozytom, zerebellarer oder zerebraler Krebs im Kindesalter, Basalzellkarzinom, Gallengangkrebs, extrahepatischer Krebs, Blasenkrebs, Knochenkrebs, Osteosarkom/malignes faseriges Histiozytom, Hirnstamm-Gliom, Hirnkrebs, Hirntumor, zerebellares Astrozytom, Hirntumor, zerebrales Astrozytom/malignes Gliom, Hirntumor, Ependymom, Hirntumor, Medulloblastom, Hirntumor, supratentorale primitive neuroectodermale Tumore, Hirntumor, Sehweg- und hypothalamisches Gliom, Brustkrebs, Bronchialadenome / Carzinoide, Burkitt-Lymphom, Carzinoidtumor, Carzinoidtumor, Magen-Darm-Trakt-Krebs, Karzinom des unbekannten Primär-, Zentralnervensystem-Lymphom, zerebellares Astrozytom, zerebrales Astrozytom/malignes Gliom, Gebärmutterhalskrebs, chronische lymphatische Leukämie, chronische myelogene Leukämie, chronische myeloproliferative Störungen, Darmkrebs, kutanes T-Zell-Lymphom, desmoplastischer kleinrundzelliger Tumor, Endometriumkrebs, Ependymom, Speiseröhrenkrebs, Ewing-Sarkom in der Ewing-Familie von Tumoren, extrakranieller Keimzelltumor, Krebs im Kindesalter, extragonadaler Keimzelltumor, extrahepatischer Gallengangkrebs, Augenkrebs, intraokulares Melanom, Augenkrebs, Retinoblastom, Gallenblasenkrebs, gastrointestinaler Karzinoid-Tumor, gastrointestinaler Magentumor (Gastrointestinal stromal tumor, GIST), Keimzelltumor: extrakranieller, extragonadaler oder ovarialer Keimzelltumor, gestationsbedingte Trophoblasttumor, Gliom des Hirnstamms, Gliom, zerebrales Astrozytom im Kindesalter, Gliom, Sehbahn- und Hypothalamusgliom im Kindesalter, Magenkarzinom, haarige Zellleukämie, Kopf- und Halskrebs, Herzkrebs, Hepatozellulärer Krebs (Leberkrebs), Hodgkin-Lymphom, Hypopharyngealkrebs, Hypothalamus- und Sehbahngliom, intraokulares Melanom, Inselzellkarzinom (endokrine Bauchspeicheldrüse), Kaposi-Sarkom, Nierenkrebs (Nierenzellkrebs), Kehlkopfkrebs, Leukämien, Leukämie, akute lymphoblastische Leukämie (auch akute lymphatische Leukämie genannt), akute myeloische Leukämie (auch akute myeloische Leukämie genannt), chronische lymphatische Leukämie (auch chronische lymphatische Leukämie genannt), chronische myeloische Leukämie (auch chronische myeloische Leukämie genannt), Haarzellen, Lippen- und Mundhöhlenkrebs, Liposarkom, Leberkrebs (primär), Lungenkrebs, nicht-kleinzelliger Lungenkrebs, kleinzelliger, Lymphome, Lymphom, AIDS-bezogenes, Lymphom, Burkitt, Lymphom, kutane T-Zell-, Lymphom, Hodgkin, Lymphome, Non-Hodgkin- (eine alte Klassifikation aller Lymphome außer Hodgkin) Lymphom, primäres Zentralnervensystem, Makroglobulinämie, Waldenströms, malignes faseriges Histiozytom des Knochens/Osteosarkoms, Medulloblastom, Melanom, Melanom, intraokular (Auge), Merkelzellkarzinom, Mesotheliom, Bösartig beim Erwachsenen, Mesotheliom, metastasierender squamöser Halskrebs mit okkultem primärem, Mundkrebs, multiples endokrines Neoplasie-Syndrom, multiples Myelom/Plasmazellneoplasma, Mycosis Fungoides, myelodysplastische Syndrome, myelodysplastische/myeloproliferative Erkrankungen, myelogene Leukämie, chronisch, myeloische Leukämie, akut beim Erwachsenen, myeloische Leukämie, akut im Kindesalter, Myelom, multipel (Krebs des Knochenmarks), myeloproliferative Störungen, Nasenhöhlen- und Nasennebenhöhlenkrebs, Nasopharynxkarzinom, Neuroblastom, Non-Hodgkin-Lymphom, nicht kleinzelliger Lungenkrebs, Mundkrebs, Oropharynx-Krebs, Osteosarkom/malignes faseriges Histiozytom des Knochens, Eierstockkrebs, Eierstockepithelkrebs (Oberflächenepithelstromatumor), Eierstockkeimzelltumor, Eierstocktumor mit niedrigem malignem Potenzial, Bauchspeicheldrüsenkrebs, Bauchspeicheldrüsenkrebs, Inselzellen, Nasennebenhöhlen- und Nasenhöhlenkrebs, Nebenschilddrüsenkrebs, Peniskrebs, Rachenkrebs, Phäochromozytom, Zirbeldrüsenkrebs, Zirbeldrüsenkeim, Pinuskeim, Pinuslastom und supratentorale primitive neuroectodermale Tumore, Hypophysenadenom, Plasmazellneoplasie/multiples Myelom, pleuropulmonales Blastom, primäres Lymphom des Zentralnervensystems, Prostatakrebs, Rektumkarzinom, Nierenzellkarzinom (Nierenkrebs), Nierenbecken und Harnleiter, Übergangszellkarzinom, Retinoblastom, Rhabdomyosarkom, Speicheldrüsenkrebs, Sarkom, Ewing-Tumorfamilie, Kaposi-Sarkom, Sarkom, Weichteilgewebe, Sarkom, uterin, Sezary-Syndrom, Hautkrebs (Nonmelanom), Hautkrebs (Melanom), Hautkarzinom, Merkelzelle, kleinzelliger Lungenkrebs, Dünndarmkrebs, Weichteilsarkom, Plattenepithelkarzinom, squamöser Halskrebs mit okkulten primären, Metastasen, Magenkrebs, supratentorieller primitiver neuroectodermaler Tumor, T-Zell-Lymphom, kutan - siehe Mycosis fungoides und Sezary-Syndrom, Hodenkrebs, Halskrebs, Thymom, Thymom und Thymokarzinom, Schilddrüsenkrebs, Schilddrüsenkrebs, Übergangszellkrebs des Nierenbeckens und Harnleiters, trophoblastischer Tumor, Harnröhren- und Nierenbecken, Übergangszellkrebs, Harnröhrenkrebs, Gebärmutterkrebs, Endometrium, uterines Sarkom, Vaginalkrebs, Sehbahn- und Hypothalamusgliom, Vulvakrebs, Waldenströms Makroglobulinämie und Wilms-Tumor (Nierenkrebs) ist.

4. Mittel zur Verwendung nach Anspruch 2, wobei der Krebs Blasenkrebs, Lungenkrebs, Brustkrebs, Melanom, Darmkrebs, Rektumkarzinom, Non-Hodgkin-Lymphom, Endometriumkrebs, Bauchspeicheldrüsenkrebs, Nierenkrebs (Nierenzellkrebs), Prostatakrebs, Leukämie, Schilddrüsenkrebs und Speiseröhrenkrebs, vorzugsweise Brustkrebs, ist.

5. Mittel zur Verwendung nach einem der Ansprüche 1 bis 4, wobei das Mittel und der Antikörper als eine kombinierte Zubereitung für eine sequentielle Verwendung vorhanden sind.

6. Mittel zur Verwendung nach einem der Ansprüche 1 bis 5, wobei das eingestellte Zeitintervall 1-2, 1-5, 1-10 oder 1-20 Tage ist.

7. Mittel zur Verwendung nach einem der Ansprüche 1 bis 6, wobei das Verfahren die folgenden Schritte umfasst:
a) Behandeln von Blut des Subjekts *ex vivo* mit dem Mittel;
b) Rückführen des behandelten Blutes an das Subjekt; und anschließend
c) Verabreichen des Antikörpers an das Subjekt.

8. Mittel zur Verwendung nach einem der Ansprüche 1 bis 7, wobei die Endoglykosidase Endoglykosidase S (EndoS) oder Endoglykosidase F3 oder Endoglykosidase Ebeta ist, oder wobei die Protease IdeS ist.

9. Mittel zur Verwendung nach Anspruch 8, wobei das Mittel EndoS oder IdeS ist.

10. Mittel zur Verwendung eines der Ansprüche 1 bis 9, wobei der Antikörper Natalizumab, Vedolizumab, Belimumab, Atacicept, Alefacept, Otelixizumab, Teplizumab, Rituximab, Ofatumumab, Ocrelizumab, Epratuzumab, Alemtuzumab, Abatacept, Eculizumab, Omalizumab, Canakinumab, Mepolizumab, Reslizumab, Tocilizumab, Ustekinumab, Briakinumab, Etanercept, Infliximab, Adalimumab, Certolizumab pegol, Golimumab, Trastuzumab, Gemtuzumab, Ozogamicin, Ibritumomab, Tiuxetan, Tositumomab, Cetuximab, Bevacizumab, Panitumumab, Denosumab, Ipilimumab, Brentuximab und Vedotin ist.

11. Mittel zur Verwendung nach Anspruch 10, wobei der Antikörper Trastuzumab ist.

## Revendications

1. Agent qui diminue la liaison au récepteur Fc par la dégradation enzymatique des anticorps sériques endogènes destiné à une utilisation dans un procédé de traitement d'une maladie, où l'agent est une endoglycosidase, une protéase ou une protéine-N-glycanase et le procédé augmente l'activité thérapeutique d'un anticorps, où le mode d'action dudit anticorps repose sur l'interaction Fc : FcR, et où le procédé comprend les étapes :
(a) d'administration dudit agent à un sujet ; et par la suite,
(b) après un intervalle de temps défini, l'administration dudit anticorps au sujet.

2. Agent destiné à une utilisation selon la revendication 1, où la maladie est un cancer, une infection ou une auto-immunité.

3. Agent destiné à une utilisation selon la revendication 2, où le cancer est la leucémie lymphoblastique aiguë, la leucémie myéloïde aiguë, le carcinome corto-surrénalien, les cancers liés au sida, le lymphome lié au sida, le cancer anal, le cancer de l'appendice, l'astrocytome du cervelet ou du cerveau chez l'enfant, le carcinome basocellulaire, le cancer du canal biliaire extra-hépatique, le cancer de la vessie, le cancer des os, l'ostéosarcome/l'histiocytome fibreux malin, le gliome du tronc cérébral, le cancer du cerveau, la tumeur cérébrale, l'astrocytome du cervelet, la tumeur cérébrale, l'astrocytome / le gliome malin cérébral, la tumeur cérébrale, l'épendymome, la tumeur cérébrale, le médulloblastome, la tumeur cérébrale, les tumeurs neuro-ectodermiques primitives supra-tentorielles, la tumeur cérébrale, le gliome hypothalamique et des voies optiques, le cancer du sein, les adénomes / les tumeurs carcinoïdes bronchiques, le lymphome de Burkitt, les tumeurs carcinoïdes, les tumeurs carcinoïdes gastro-intestinales, le carcinome primitif inconnu, le lymphome du système nerveux central, l'astrocytome du cervelet, l'astrocytome / le gliome malin cérébral, le cancer du col de l'utérus, la leucémie lymphoïde chronique, les troubles myéloprolifératifs chroniques de la leucémie myéloïde chronique, le cancer du côlon, le lymphome cutané à cellules T, la tumeur desmoplastique à petites cellules rondes, le cancer de l'endomètre, l'épendymome, le cancer de l'oesophage, le sarcome d'Ewing de la famille des tumeurs d'Ewing, les tumeurs cellulaires germinales extra-crâniennes chez l'enfant, les tumeurs cellulaires germinales extra-gonadiques, le cancer du canal biliaire extra-hépatique, le cancer de l'oeil, le mélanome intraoculaire, le cancer de l'oeil, le rétinoblastome, le cancer de la vésicule biliaire, le cancer gastrique (de l'estomac), la tumeur carcinoïde gastro-intestinale, la tumeur stromale gastro-intestinale (GIST), les tumeurs cellulaires germinales : extra-crâniennes, extra-gonadiques ou ovariennes, la tumeur trophoblastique gestationnelle, le gliome du tronc cérébral, le gliome, l'astrocytome cérébral chez l'enfant, le gliome hypothalamique et des voies optiques chez l'enfant, les tumeurs carcinoïdes gastriques, la leucémie à tricho-leucocytes, le cancer des voies aéro-digestives supérieures, le cancer du coeur, le cancer hépatocellulaire (du foie), le lymphome de Hodgkin, le cancer de l'hypo pharynx, le gliome hypothalamique et des voies optiques, le mélanome intraoculaire, le carcinome des cellules des îlots (pancréas endocrinien), le sarcome de Kaposi, le cancer du rein (cancer des cellules rénales), le cancer du larynx, les leucémies, la leucémie lymphoblastique aiguë (également appelée leucémie lymphoïde aiguë), la leucémie myéloïde aiguë (également appelée leucémie myéloblastique aiguë), la leucémie lymphoïde chronique (également appelée leucémie lymphocytaire chronique), la leucémie, myéloïde chronique (également appelée leucémie myéloblastique chronique), la leucémie à tricho-leucocytes, le cancer de la lèvre et de la cavité buccale, le liposarcome, le cancer du foie (primitif), le cancer du poumon non à petites cellules, le cancer du poumon à petites cellules, les lymphomes, le lymphome lié au sida, le lymphome de Burkitt, le lymphome cutané à cellules T, le lymphome de Hodgkin, les lymphomes non hodgkiniens (une ancienne classification de tous les lymphomes à l'exception de celui de Hodgkin), le lymphome primitif du système nerveux central, la macroglobulinémie de Waldenstrôm, l'histiocytome fibreux malin des os / l'ostéosarcome, le médulloblastome, le mélanome, le mélanome intraoculaire (de l'oeil), le carcinome à cellules de Merkel, le mésothéliome malin chez l'adulte, le mésothéliome, le cancer squameux métastatique du cou avec primitif occulte, le cancer de la bouche, le syndrome de l'adénomatose poly endocrinienne, le myélome multiple / néoplasme des plasmocytes, le mycosis fongoïde, les syndromes myélodysplasiques, les maladies myélodysplasiques / myéloprolifératives, la leucémie myéloïde chronique, la leucémie myéloïde aiguë chez l'adulte, la leucémie myéloïde aiguë chez l'enfant, le myélome multiple (cancer de la moelle osseuse), les troubles myéloprolifératifs, le cancer du sinus paranasal et de la cavité nasale, le carcinome du nasopharynx, le neuroblastome, le lymphome non hodgkinien, le cancer du poumon non à petites cellules, le cancer buccal, le cancer de l'oropharynx, l'ostéosarcome/l'histiocytome fibreux malin des os, le cancer de l'ovaire, le cancer épithélial de l'ovaire (tumeur épithélio-stromale de la surface), la tumeur des cellules germinales de l'ovaire, la tumeur à faible potentiel de malignité de l'ovaire, le cancer du pancréas, le cancer du pancréas, des cellules des îlots, le cancer de la cavité nasale et du sinus paranasal, le cancer de la parathyroïde, le cancer du pénis, le cancer du pharynx, le phéochromocytome, l'astrocytome pinéal, le germinome pinéal, le pinéaloblastome et les tumeurs neuro-ectodermiques primitives supra-tentorielles, l'adénome hypophysaire, la néoplasie à plasmocytes / le myélome multiple, le blastome pleuro-pulmonaire, le lymphome primitif du système nerveux central, le cancer de la prostate, le cancer rectal, le carcinome à cellules rénales (le cancer du rein), du bassinet du rein et de l'uretère, le cancer à cellules transitionnelles, le rétinoblastome, le rhabdomyosarcome, le cancer de la glande salivaire, le sarcome de la famille des tumeurs d'Ewing, le sarcome de Kaposi, le sarcome des tissus mous, le sarcome de l'utérus, le syndrome de Sézary, le cancer de la peau (non mélanome), le cancer de la peau (mélanome), le carcinome de la peau à cellules de Merkel, le cancer du poumon à petites cellules, le cancer de l'intestin grêle, le sarcome des tissus mous, le carcinome épidermoïde, le cancer squameux du cou avec primitif occulte, métastasique, le cancer de l'estomac, les tumeurs neuro-ectodermiques primitives supra-tentorielles, le lymphome à cellules T, cutané - voir le mycosis fongoïde et le syndrome de Sézary, le cancer des testicules, le cancer de la gorge, le thymome, le thymome et le carcinome thymique, le cancer de la thyroïde, le cancer de la thyroïde, le cancer à cellules transitionnelles du bassinet du rein et de l'uretère, la tumeur trophoblastique, uretère et bassinet du rein, le cancer à cellules transitionnelles, le cancer urétral, le cancer de l'utérus, de l'endomètre, le sarcome de l'utérus, le cancer du vagin, le gliome hypothalamique et des voies optiques, le cancer de la vulve, la macroglobulinémie de Waldenstrôm et la tumeur de Wilms (le cancer du rein).

4. Agent destiné à une utilisation selon la revendication 2, où le cancer est le cancer de la vessie, le cancer du poumon, le cancer du sein, le mélanome, le cancer du côlon, le cancer rectal, le lymphome non hodgkinien, le cancer de l'endomètre, le cancer du pancréas, le cancer du rein (des cellules rénales), le cancer de la prostate, la leucémie, le cancer de la thyroïde et le cancer de l'oesophage, de préférence le cancer du sein.

5. Agent destiné à une utilisation selon l'une quelconque des revendications 1 à 4, où l'agent et l'anticorps sont présents en tant que préparation combinée destinée à une utilisation séquentielle.

6. Agent destiné à une utilisation selon l'une quelconque des revendications 1 à 5, où l'intervalle de temps défini est de 1 à 2, de 1 à 5, de 1 à 10 ou de 1 à 20 jours.

7. Agent destiné à une utilisation selon l'une quelconque des revendications 1 à 6, où le procédé comprend les étapes :
(a) de traitement *ex vivo* du sang provenant du sujet avec l'agent ;
(b) de restitution au sujet du sang traité ; et ensuite
(c) d'administration dudit anticorps au sujet.

8. Agent destiné à une utilisation selon l'une quelconque des revendications 1 à 7, où l'endoglycosidase est l'endoglycosidase S (EndoS) ou l'endoglycosidase F3, ou l'endoglycosidase Ebêta, ou où la protéase est l'IdeS.

9. Agent destiné à une utilisation selon la revendication 8, où l'agent est l'EndoS ou l'IdeS.

10. Agent destiné à une utilisation selon l'une quelconque des revendications 1 à 9, où ledit anticorps est le natalizumab, le védolizumab, le belimumab, l'atacicept, l'alefacept, l'otelixizumab, le teplizumab, le rituximab, l'ofatumumab, l'ocrélizumab, l'epratuzumab, l'alemtuzumab, l'abatacept, l'éculizumab, l'omalizumab, le canakinumab, le mépolizumab, le reslizumab, le tocilizumab, l'ustékinumab, le briakinumab, l'étanercept, l'infliximab, l'adalimumab, le certolizumab pegol, le golimumab, le trastuzumab, le gemtuzumab, ozogamicin, l'ibritumomab, le tiuxétan, le tositumomab, le cétuximab, le bévacizumab, le panitumumab, le dénosumab, l'ipilimumab, et le brentuximab et védotine.

11. Agent destiné à une utilisation selon la revendication 10, où ledit anticorps est le trastuzumab.
